# EUROPEAN PATENT APPLICATION

(11) **EP 4 273 236 A1**
(43) Date of publication of application: **08.11.2023**
(21) Application number: 21914357.5
(22) Date of filing: 28.12.2021
(51) Int. Cl.: C12N 7/02, A61K 48/00, C12N 7/00, C12N 15/867

(54) **HEK293T CELL STRAIN HAVING HIGH DISPERSIBILITY AND SCREENING METHOD THEREFOR**

(30) Priority: 29.12.2020 CN 202011592436
(71) Applicant: Jiangsu Genscript Probio Biotech Co., Ltd., Zhenjiang, Jiangsu 212000 (CN)
(72) Inventor: GUO, Chen, Zhenjiang, Jiangsu 212000 (CN); XIE, Shenghua, Zhenjiang, Jiangsu 212000 (CN); XUAN, Chunling, Zhenjiang, Jiangsu 212000 (CN); HUI, Erjing, Zhenjiang, Jiangsu 212000 (CN); DING, Rongna, Zhenjiang, Jiangsu 212000 (CN)
(74) Representative: Pharma Patents International AG
(86) International application number: PCT/CN2021/141958
(87) International publication number: WO 2022/143623

(57) **Abstract**

Provided are a HEK293T cell strain having high dispersibility and a screening method therefor. Specifically, the present application relates to a method for screening a HEK293T cell strain suitable for serum-free suspension culture, a cell strain screened by using the method, and a method for producing a viral vector by using the cell strain.

## Description

### Related Application and Incorporated By Reference

All documents cited or mentioned in the present application (including but not limited to all literatures, patents, and published patent applications cited herein) ("the documents cited in the present application"), all documents cited or mentioned in the documents cited in the present application, and the manufacturer's manuals, instructions, product specifications and product pages of any products mentioned in the present application or in any documents cited in the present application are incorporated by reference into the present application, may be adopted in the practice of the present invention. More specifically, all references are incorporated by reference into the present application as if each individual document is incorporated by reference. Any Genbank sequences mentioned herein are incorporated by reference into the present application.

Citation of any document in the present application does not constitute an admission that such document is prior art to the present application.

### Field of the Invention

The present application relates to a method for screening a HEK293T cell strain suitable for serum-free suspension culture, especially suitable for serum-free suspension culture and having high dispersibility, and a HEK293T cell strain screened by using the method, especially PowerS^{™}-293T with a deposit number of CGMCC No.: 21100. The present application also relates to a method for producing a viral vector using the screened cell strain, especially PowerS^{™}-293T.

### Background Art

Gene therapy and cell therapy have entered a stage of rapid development and become the most promising development direction of medical care for life. The main goal of gene therapy is to introduce an exogenous gene into a target cell or tissue, replace, compensate, block, and modify a specific gene to achieve the purpose of treating a disease. In gene therapy, 70%-80% of therapeutic regimens are accomplished by a viral vector. The viral vector is a key carrier to deliver a therapeutic exogenous gene to a target site, and can be used as a drug for direct injection or as an important raw material for drug production. The viral vector currently used for introduction of an exogenous gene into a target cell is mainly derived from a viral vector such as a retrovirus vector, an adenovirus vector, and an adeno-associated virus vector. Lentivirus vector is a kind of retroviral vector, which is a high-efficiency vector developed on the basis of human immunodeficiency virus type I (HIV-1). Generally, there are two methods for the production of a lentiviral vector: One is to use a cell strain that stably expresses an element of the lentiviral packaging system; the other is to use the transient transfection method. The packaging method using transient transfection is simple and time-saving, and in the transient transfection vector system, many transgenes and messenger glycoproteins can be easily replaced, so the method of producing a lentiviral vector using transient transfection is still the most commonly used.

A HEK293 cells, also known as human embryonic kidney cell 293, is the most commonly used cell for the production of a retrovirus and other viral vectors. This cell strain derived from a human embryonic kidney cell has the characteristics of high transfection efficiency and easy culture. The HEK293T cell strain is formed after a HEK293 cell is transfected with the SV40 large T antigen gene. The HEK293T cell can significantly amplify the plasmid containing the SV40 replication origin and promote protein expression, so it is used for the expression of various genes and the production of proteins.

At present, most lentiviruses are produced by adding fetal bovine serum for adherent culture of a HEK293-derived cell strain (such as 293T and 293E) in a square bottle, a plate and other systems, and then the virus is obtained by transient transfection and packaging with multiple plasmids. For R&D enterprises of cell and gene therapy, the demand for viral vectors is limited during preclinical research, IND declaration and clinical trials, so almost all of the enterprises use transient transfection of adherent HEK293T cells to produce lentiviral vectors. The adherent culture of HEK293T cells is usually in the form of a cell factory, which mainly relies on increasing the number of culture units to increase the amount of cells. Therefore, when a large number of viral vectors is required, it often needs to occupy a large space, and the production labour is also large.

To obtain large growth areas while reducing labour, one approach is to adapt a lentivirus-producing cell strain to suspension culture. Compared with adherent culture, suspension culture can greatly increase cell density. In addition, serum-free medium can be used to cultivate cells, reduce potential immunogenic substance contamination and animal-derived components in the final product, and simplify the downstream purification process, thereby greatly promoting the transformation of the product to clinical grade. Scale-up of suspension culture can also reduce batch-to-batch variability compared to the original adherent culture by increasing the number of culture units. However, during the serum-free suspension adaption of HEK293T cells, there is a problem that the cells are easy to clump. Cell clumping prevent many of the plasmids used for vector production from transfecting cells, and cells in the middle of large clumps die from lack of nutrients. At present, the method of improving cell clumping is mainly by adding an anti-clumping agent. The addition of the anti-clumping agent may significantly reduce the transfection efficiency, resulting in cells that cannot be transfected with packaged lentiviruses.

### Summary of the Invention

Through extensive experiments, the inventors of the present application found a method for screening a HEK293T cell strain suitable for serum-free suspension culture. By this method, a cell strain having high dispersibility and high proliferation density can be screened out.

Thus, in one aspect, the present application provides a method for screening a HEK293T cell strain suitable for serum-free suspension culture, comprising:
i) taking a HEK293T cell adherently cultured in a serum-containing medium and placing the cell in a serum-free medium for suspension culture, and
ii) selecting a monoclonal cell strain suitable for serum-free suspension culture.

The serum-containing medium in step i) may contain 10% serum, such as 10% fetal bovine serum.

The suspension culture in step i) may include shaking culture at 37°C and 8% CO₂.

The suspension culture in step i) may include cell passaging.

The serum-free medium can be selected from LV-MAX production medium (Gibco, A35834-01), Expi293 expression medium (Gibco, A14351-01), FreeStyle^{™} 293 expression medium (Gibco, 12338-018), CD293 AGT medium (Gibco, 11913-019), EX-CELL^{®}293 serum-free medium (Sigma, 14571C-1000mL), BanlanCD HEK293 medium (Irvine, 91165), Pro293s-CDM serum-free medium (Lonza, 12-765Q), OPM-293 CD03 medium (Shanghai OPMBiosciences Co., Ltd., 81070-001) and Celkey CD HEK293 medium (JSBiosciences, 10804-19008). The serum-free medium is preferably LV-MAX production medium, Expi293 expression medium and OPM-293 CD03 medium.

Step ii) may include selecting a non-clumping monoclonal cell strain suitable for serum-free suspension culture.

Step ii) may include culturing the cell in a semi-solid medium, selecting a single cell from the semi-solid medium, and performing suspension culture on the cell.

The suspension culture of the selected cell in step ii) may include shaking culture at 37°C and 8% CO₂. The suspension culture of the selected cell in step ii) may include cell passaging, for example, more than 3 passages.

The semi-solid medium may be a methylcellulose-based medium supplemented with a screening medium.

During the implementation of this method, no anti-clumping agent needs to be added.

In the method of the present application, 1) the cells adherently cultured in the serum-containing medium are directly subjected to serum-free suspension culture, and there is no step of progressive serum decreasing, and the screening process can be can shortened; 2) the serum-free medium can be used to culture the cells, reduce potential immunogenic substance contamination and animal-derived components, and simplify the downstream purification process; 3) the serum-free medium selected from LV-MAX production medium, Expi293 expression medium and OPM-293 CD03 medium is used, and is more conducive to screening the HEK293T cell strain suitable for suspension culture, that is, with high proliferation density and high dispersibility; 4) the use of the anti-clumping agent is avoided, and the transfection efficiency of the cells is not affected; and 5) the use of the semi-solid medium facilitates the screening of qualified single clones.

The present application also encompasses the use of a medium selected from LV-MAX production medium (Gibco, A35834-01), Expi293 expression medium (Gibco, A14351-01) and OPM-293 CD03 medium (Shanghai OPM Biosciences Co., Ltd., 81070-001) in screening a HEK293T cell strain with high dispersibility and high proliferation density.

In another aspect, the present application relates to the HEK293T cell strain screened by using the above method.

In particular, the present application provides a novel human embryonic kidney HEK293T cell strain PowerS^{™}-293T, which was deposited at the China General Microbiological Culture Collection Centre (CGMCC) on December 7, 2020 according to the Budapest Treaty and has a deposit number of CGMCC No.: 21100.

The cell strain can grow in suspension culture in a serum-free medium. The serum-free medium can be selected from LV-MAX production medium, Expi293 expression medium and OPM-293 CD03 medium.

Cell clumping cannot appear during the suspension culture of the cell strain. This cell strain can be used to produce a viral vector.

PowerS^{™}-293T cell strain can grow in suspension culture in the serum-free medium, with high proliferation density, good viability, and no cell clumping, that is, with high dispersibility. Therefore, no additional anti-clumping agent is needed in the suspension culture process. Compared with Gibco's CTS^{™} virus production cells (Pub. No. MAN0018590), PowerS^{™}-293T has a shorter cell doubling time and produces more viral vectors after transfection with viral plasmids.

In another aspect, the present application relates to the use of the screened HEK293T cell strain in the preparation of a viral vector.

Specifically, the present application provides a method for preparing a viral vector using the cell strain screened by using the present application, including PowerS^{™}-293T, comprising:
i) culturing the cell strain;
ii) introducing a virus vector expression system into the cell strain;
iii) culturing the cell strain under a condition favourable for the production of the viral vector; and
vi) collecting the viral vector.

The method may also comprise determining the titre of the viral vector after step vi).

Step i) may include suspension culture of the cell strain. In one embodiment, the cell strain is cultured with shaking in serum-free medium under the condition of 37°C and 8% °C. The serum-free medium can be selected from LV-MAX production medium, Expi293 expression medium and OPM-293 CD03 medium.

Step i) may also include passaging of the cell strain.

Step ii) may include introducing the viral expression system into the cell strain via a physical or chemical transfection. The physical transfection can include electroporation, microinjection, gene guns, *etc.* The chemical transfection method can include calcium phosphate co-precipitation, polyethyleneimine (PEI)-mediated transfection, Lipofectamine (Invitrogen)-mediated transfection, *etc.*

The viral vector expression system can comprise a packaging nucleic acid vector and an envelope nucleic acid vector. The viral vector expression system can also comprise an accessory nucleic acid vector. The viral vector expression system can also comprise a nucleic acid vector containing a heterologous nucleic acid, such as a transfer nucleic acid vector containing a heterologous nucleic acid. These nucleic acid vectors can be plasmids.

The heterologous nucleic acid is no greater than 4 kb in length. The heterologous nucleic acid may encode a polypeptide or a protein such as a chimeric antigen receptor (CAR), or a nucleic acid such as siRNA. In one embodiment, the heterologous nucleic acid may encode a CAR, such as a CAR targeting CD19, CD20, CD22, CD30, CD33, or CD269 (BCMA). In one embodiment, the CAR encoded by the heterologous nucleic acid can target an antigen involved in growth and differentiation signalling, including a carcinoembryonic antigen (CEA), an epidermal growth factor receptor (EGFR, HER2), a tyrosine kinase receptor (EPHA2), *etc.* In one embodiment, the CAR encoded by the heterologous nucleic acid can target an antigen involved in tumour angiogenesis, including vascular endothelial growth factor (VEGF), VEGF receptor (VEGFR), *etc.* In one embodiment, the CAR encoded by the heterologous nucleic acid can target a tumour interstitial and an extracellular matrix antigen of the tumour support structure, including fibroblast activation protein (FAP), tenascin protein, *etc.* The plasmids containing the heterologous nucleic acid can include pCDH, pCMV, *etc.*

The viral vector may be a retroviral vector, an adenoviral vector or an adeno-associated viral vector. The retroviral vector may be a lentiviral vector, an alpha-retroviral vector, a gamma-retroviral vector, or a foamy retroviral vector. In one embodiment, the retroviral vector is a lentiviral vector selected from HIV-1, HIV-2, SIV, FIN, EIAV and VISNA. In one embodiment, the lentiviral vector may be an HIV-1 vector. In one embodiment, the viral vector is an adeno-associated viral vector. In one embodiment, the adeno-associated viral vector is selected from AAV2, AAV5, AAV8, AAV9 and AAV10.

The retroviral vector expression system may comprise, a vector containing retroviral structural protein gag-pol gene, and a nucleic acid vector containing env gene or a functional substitute thereof. The env gene or the functional substitute thereof can encode vesicular stomatitis virus glycoprotein (VSV-G) or a derivative protein thereof. The retroviral vector expression system may also comprise a nucleic acid vector containing an accessory gene rev or a similar gene. The retroviral vector expression system can also comprise a nucleic acid vector containing a heterologous nucleic acid, such as a transfer nucleic acid vector containing a heterologous nucleic acid.

In one embodiment, the method is used to prepare a lentiviral vector, and comprises introducing three nucleic acid vectors into the cell strain. The three nucleic acid vectors comprise HIV-1 gag-pol-rev gene, a nucleic acid encoding vesicular stomatitis virus glycoprotein (VSV-G) or a derivative protein thereof, and a heterologous nucleic acid, respectively.

In one embodiment, the method is used to prepare a lentiviral vector, and comprises introducing four nucleic acid vectors into the cell strain. The four nucleic acid vectors comprise HIV-1 gag-pol-rev gene, a HIV-1 rev gene, a nucleic acid encoding vesicular stomatitis virus glycoprotein (VSV-G) or a derivative protein thereof, and a heterologous nucleic acid, respectively. The nucleic acid vector containing the heterologous nucleic acid may contain a self-inactivating lentiviral long terminal repeat.

Step iii) may include suspension culture of the cell strain. In one embodiment, the cell strain is cultured with shaking in serum-free medium under the condition of 37°C and 8%°C. The serum-free medium can be selected from LV-MAX production medium, Expi293 expression medium and OPM-293 CD03 medium.

Step iii) may include passaging the cells.

Other features and advantages of the present disclosure will be apparent from the following detailed descriptions and examples, which should not be viewed as limiting. All citations, GenBank accession numbers, patents and published patent applications cited in the present application are expressly incorporated herein by reference.

It should be noted that in the present application, especially in the claims, terms such as "comprise", "include", *etc.*, terms such as "essentially consist of" allow the presence of elements not explicitly stated. The aspects and embodiments of the present invention described herein include aspects and embodiments involving "comprise", "consist of", and "essentially consist of".

As used in the present application and appended claims, the singular forms "a," "an," and "the" include plural referents, unless the context clearly dictates otherwise. Therefore, for example, reference to "a molecule" optionally includes a combination of two or more of such molecules, and the like.

As used the present application, the term "about" refers to a conventional error range of the corresponding numerical value easily known by those skilled in the art. Reference herein to "about" a certain value or a parameter includes (and describes) the embodiment involving the value or parameter itself.

### Brief Description of the Drawings

The following detailed description is given by way of example and is not intended to limit the present invention to the specific embodiments, and can be better understood in conjunction with the accompanying drawings.
FIG. 1 shows the morphology of cells adapted in different media.
FIG. 2 shows the growth curves of cells adapted in three serum-free media.
FIG. 3 shows the morphology of monoclonal cells adapted in three serum-free media.
FIG. 4 shows the cell morphology (A) and growth curve (B) of the cell strain PowerS^{™}-293T.

Deposit instruction: The novel human embryonic kidney HEK293T cell strain PowerS^{™}-293T involved in the present application was deposited at the China General Microbiological Culture Collection Centre (CGMCC) on December 7, 2020 according to the Budapest Treaty with a deposit number of CGMCC No.: 21100.

### Detailed Description of Embodiments

Unless defined otherwise, the technical and scientific terms used herein have the same meaning as commonly understood by a person skilled in the art to which the present invention belongs.

The present application provides a method for screening a cell strain suitable for serum-free suspension culture, and the screened cell strain. The cell strain has high dispersibility and high proliferation density, and can be used to produce a viral particle vector with high-titre, especially a retroviral vector, particularly a lentiviral vector particle.

### Viral vector

The viral vector is a tool used by molecular biologists to deliver a genetic material to a cell, carries an exogenous gene and can be packaged into a virus particle to mediate the transfer and expression of the exogenous gene, and are not pathogenic to the body. "Heterologous nucleic acid", "heterologous gene", "exogenous nucleic acid" or "exogenous gene" in the present application refers to a nucleic acid or gene that does not naturally occur in a virus. Generally, the heterologous nucleic acid or exogenous gene contains an open reading frame encoding a protein of interest or an untranslated RNA.

The terms "viral vector", "viral particle" or "viral vector particle" are used interchangeably herein.

Known viral vectors include retroviral vectors, adenoviral vectors, adeno-associated viral (AAV) vectors, herpes virus, *etc.*

### Retroviral

The retroviral vector is the most used viral vector in human gene therapy research. Retroviruses are a family of viruses that contain a pseudodiploid single-stranded RNA genome that encodes a reverse transcriptase that produces DNA from the RNA genome and the DNA can then be inserted into host cell DNA. All retroviruses contain at least three genes, *gag, pol,* and *env,* which encode structural proteins and enzymes (reverse transcriptase, RNase H, integrase, and protease) that are essential for viral replication.

For a better understanding of retroviruses, the nucleic acid sequences common to all retroviruses are first explained as follows.

Long terminal repeat (LTR): The basic structure of a retroviral genome contains 5'-LTR and 3'-LTR, and a gene required for retrovirus production located therebetween or therein. LTRs are required for retroviral integration and transcription. They can also act as promoter sequences to control the expression of retroviral genes *(i.e.,* they are *cis*-acting genes). The LTR consists of three divided subregions U3, R, and U5: U3 is derived from a sequence unique to the 3' end of RNA; R is derived from the sequence repeated at both ends of the RNA; U5 is derived from a sequence unique to the 5' end of RNA. To address the safety concerns associated with generating replication-competent viruses, a self-inactivating (SIN) vector has been developed by deleting a segment in the U3 region of the 3' LTR, the U3 region includes a TATA box and binding sites of transcription factors Sp1 and NF-κB (Miyoshi H et al., (1998) Development of a self-inactivating lentivirus vector. J Virol. 72(10):8150-8157). Upon reverse transcription and integration into infected cells, this deletion is transferred into the 5' LTR, resulting in transcriptional inactivation of the LTR.

ψ: Encapsidation of retroviral RNA occurs by means of the ψ (psi) sequence located at the 5' end of the retroviral genome. It is well known in the art that sequence downstream of the psi sequence and extending into the gag coding region is involved in efficient retroviral vector production (Yan Cui et al., (1999) Contribution of viral splice sites and cis-regulatory elements to lentivirus vector function. J. Virol. 73(7):6171-6176).

Primer binding site (PBS): The retroviral genome contains PBS following the U5 region of the 5'-LTR. This site binds the tRNA primer required to initiate reverse transcription.

PPT: The retroviral genome contains a short stretch of purines called the polypurine fragment (PPT) near the 3' end of the retroviral genome. These PPTs function as RNA primers for positive-strand DNA synthesis during reverse transcription. Complex retroviruses, such as HIV-1, contain a second, more centrally located PPT *(i.e.,* the central polypurine domain (cPPT)), which provides a second site for initiation of DNA synthesis. Retroviral vectors encoding cPPT have been shown to have enhanced transduction and transgene expression (Barry SC et al., (2001) Lentivirus vectors encoding both central polypurine tract and posttranscriptional regulatory element provide enhanced transduction and transgene expression. Hum Gene Ther. 12(9):1103-1108).

The genomic structures of the above non-coding regions are well known to those skilled in the art. For example, details on the genome structures of non-coding regions in HIV-1 can be found from NCBI Genbank under Genome Accession No. AF033819, or Genome Accession No. K03455 for HIV-1 HXB2 (a commonly used HIV-1 reference strain).

Gag/pol: Expression of gag and pol genes is dependent on a translational frameshift between gag and pol. Both are polyproteins that are cleaved during maturation. The main structural matrix, capsid and nucleocapsid proteins of retroviral vectors are encoded by gag. The pol gene encodes enzymes related to viral replication, including: i) a reverse transcriptase, which is essential for the reverse transcription of retroviral RNA genome to double-stranded DNA; ii) an integrase, which integrates the retroviral DNA genome into the host cell chromosome; and iii) a protease, which cleaves the synthesized polyprotein to produce the mature and functional protein of the retrovirus. In one embodiment, the retroviral nucleic acid sequence encoding the gag and pol proteins is derived from the HIV-1 HXB2 sequence, which is available from Genome Accession No. K03455, for example from base pairs 790-5105.

Env: The *env* ("envelope") gene encodes the surface and transmembrane components of the retroviral envelope (such as the glycoproteins gp120 and gp41 of HIV-1) and is involved in retrovirus-cell membrane fusion. In order to broaden the tissue transfection of retroviral vectors, retroviral vectors can be pseudotyped with envelope proteins from another virus. Pseudotyping refers to the extension or alteration of the host cell range of retroviral vectors (including lentiviral vectors) by altering the glycoprotein (GP) on the retroviral vector particle, for example by using GPs obtained from or derived from other enveloped viruses or using synthetic/artificial GPs. The most commonly used glycoprotein for pseudotyping retroviral vectors is vesicular stomatitis virus GP (VSVg) due to its wide range of transfection and high vector particle stability. However, those skilled in the art will understand that other glycoproteins can be used for pseudotyping (Cronin J et al., (2005) Altering the tropism of lentiviral vectors through pseudotyping. Curr Gene Ther. 5(4):387-398). The choice of viruses for pseudotyping may also depend on the type of cells and/or organs to be targeted. The env protein or a functional substitute thereof may be obtained or derived from a virus selected from: Vesiculovirus (for example, vesicular stomatitis virus), Lyssavirus (for example, rabies virus, and Mokola virus), Arenaviruses (for example, lymphocytic choriomeningitis virus (LCMV)), Alphaviruse (for example, Ross River virus (RRV), Sindbis virus, semliki forest virus (SFV), venezuelan equine encephalitis virus), filoviruses (for example, Reston strain of Ebola virus, Zaire strain of Ebola virus, and Lassa virus), Alpharetrovirus (for example, avian leukosis virus (ALV)), Betaretrovirus (for example, sheep lung adenoma retrovirus (JSRV)), Gammaretrovirus (for example, Moloney murine leukemia virus (MLV), Gibbon leukemia virus (GALV), Feline endogenous retrovirus (RD114)), Deltaretrovirus (for example human T-lymphotropic virus type 1 (HTLV-1)), Spumavirus (for example, human foamy virus), Lentivirus (for example Maedi-Visna virus (MVV)), coronaviruses (for example SARS-CoV), respiratory viruses (for example, Sendai virus, respiratory syncytial virus (RSV)), hepatitis C viruses (for example hepatitis C virus (HCV)), influenza viruses (for example influenza A virus) and nuclear polyhedrosis viruses (for example Autographa californica multiple nuclear polyhedrosis virus (AcMNPV)). In one embodiment, the env protein or the functional substitute thereof is obtained from or derived from vesicular stomatitis virus. In this embodiment, the vesicular stomatitis virus glycoprotein (VSVg) protein can be used, and allows the retroviral particle to infect a wider range of host cells and eliminates the chance of recombinant production of the wild-type envelope protein. In another embodiment, the retroviral nucleic acid sequence encoding the env protein or a functional substitute thereof is derived from the sequence at Genome Accession No. J02428.1, for example from base pairs 3071 to 4720.

The structural genes described herein are common to all retroviruses. Other accessory genes may be present in different types of retroviruses. For example, lentiviruses, such as HIV-1, contain six additional accessory genes called *rev*, *vif*, *vpu*, *vpr*, *nef*, and *tat.* Other retroviruses may have accessory genes similar to those described herein, however, they may not always be given the same names as in the literature. For example, Tomonaga and Mikami described various retroviral accessory genes (Tomonaga K, Mikami T. (1996) Molecular biology of the feline immunodeficiency virus accessory genes. J Gen Virol 77(Pt 8): 1611-1621).

Rev: The accessory gene *rev* ("regulator of the virion") encodes an accessory protein that binds to the Rev response element (RRE) and facilitates the export of retroviral transcripts. The protein product of this gene allows the export of retroviral mRNA fragments containing the Rev response element (RRE) from the nucleus to the cytoplasm. It is predicted that the RRE sequence forms a complex folding structure. This specific role of rev reflects the tight coupling of the steps of splicing and nuclear export. The RRE sequence may be derived from the HIV-1 HXB2 sequence, which is available at Genome Accession No. K03455, for example from base pairs 7622 to 8479 or 7769 to 8146, especially base pairs 7622 to 8479.

Rev binds to the RRE and promotes the export of either singly spliced (*env*, *vif*, *vpr*, and *vpu*) or non-spliced (*gag*, *pol*, and genomic RNA) viral transcripts, leading to downstream events such as gene translation and packaging (Suhasini M, Reddy TR. (2009) Cellular proteins and HIV-1 Rev function. Curr HIV Res. 7(1):91-100). In one embodiment, the viral expression system comprises the accessory gene *rev* or a gene similar to it *(i.e.,* from other retroviruses or functionally similar systems). Inclusion of the *rev* gene ensures efficient export of RNA transcripts of the retroviral vector genome from the nucleus to the cytoplasm, especially if RRE element is also included in the transcripts to be transported. The *rev* gene may have at least 60% sequence identity, such as at least 70% sequence identity, to base pairs 970 to 1320 of Genome Accession No. M11840 *(i.e.,* HIV-1 clone 12 cDNA, HIVPCV12 locus). In an alternative embodiment, the *rev* gene has at least 60% sequence identity, such as at least 70%, 80%, 90% or 100% sequence identity with base pairs 5970 to 6040 and 8379 to 8653 of Genome Accession No. K03455.1 (*i.e.*, human immunodeficiency virus type 1, HXB2).

Accessory genes are thought to play a role in retroviral replication and pathogenesis, so many current viral vector production systems do not include some of these genes. The commonly existing *rev* is an exception, or a system similar to the rev/RRE system can be used. In one embodiment, the nucleic acid sequence encoding one or more accessory genes *vpr*, *vif*, *vpu*, *tat* and *nef* or similar accessory genes in the viral vector can be disrupted such that the accessory genes are removed from the RNA genome of retroviral vector particles or cannot encode functional accessory proteins. In another embodiment, at least two or more, three or more, four or more or all of the accessory genes *vpr*, *vif*, *vpu*, *tat* and *nef* or similar accessory genes are disrupted such that the accessory genes are removed from the RNA genome of retroviral vector particles or cannot encode functional accessory proteins. Removal of functional accessory genes may not require removal of the entire gene; it is sufficient to remove part of the gene or disrupt the gene.

It will be appreciated that the nucleic acid sequence encoding the replication-deficient retroviral vector particle may be identical to or derived from the wild-type gene of the retrovirus on which the retroviral vector particle is based, *i.e.,* the sequence may be a genetically or otherwise altered version of the sequence contained in the wild-type virus. Thus, a retroviral gene introduced into a nucleic acid vector or a host cell genome may also refer to a codon-optimized version of the wild-type gene.

### Lentiviral vector

As a kind of retroviral vector, the lentiviral vector is a gene therapy vector developed on the basis of HIV-1, which can introduce a target gene into an animal and human primary cell or cell strain. The lentiviral genome is positive-strand RNA. After entering the cell, the lentiviral genome is reversed into DNA by a reverse transcriptase carried by itself in the cytoplasm, forming a pre-integration DNA complex. After entering the cell nucleus, the DNA is integrated into the cell genome. The integrated DNA is transcribed into mRNA, which returns to the cytoplasm to express the target protein. Lentivirus-mediated gene expression is sustained and stable due to the integration of the gene of interest into the host cell genome and division as the cell genome divides. Additionally, lentiviruses efficiently infect and integrate into non-dividing cells. The above characteristics make lentiviral vectors have extra advantages compared with other viral vectors, such as non-integrating adenoviral vectors, adeno-associated viral vectors with low integration rates, and traditional retroviral vectors that only integrate into dividing cells.

The lentiviral vector is based on the lentiviral genome, and many sequence structures related to viral activity are removed to make the vector biologically safe. Then, the gene sequence of interest and expression structure needed for clinical use or research are introduced into this genome skeleton to prepare the vector. The current lentiviral vector contains all the genetic information required for packaging, transfection, and stable integration, and is the main component of the lentiviral vector system. With the assistance of lentiviral packaging plasmids and cell strains, the lentiviral vectors carrying exogenous genes are packaged into infectious virus particles, which can be collected and concentrated to directly infect host cells or animal models to achieve the expression of the exogenous genes in cells or living tissues. Studies have shown that lentiviral vectors can effectively infect neuronal cells, liver cells, cardiomyocytes, tumour cells, endothelial cells, stem cells and other types of cells, and mediate long-term expression of target genes. At present, the lentivirus is also widely used in the research of expressing RNAi. Lentiviral vectors can produce high-titre lentiviruses expressing shRNA, and the high-titre lentiviruses express shRNA in periodic and non-periodic cells, stem cells, fertilized eggs, and differentiated offspring cells, thereby realizing the specific and stable functional silencing of gene expression in various types of cells and transgenic mice, and providing the possibility for quickly and efficiently studying gene function in primary human and animal cell tissues and producing animals with reduced expression of specific genes.

Early lentiviral vectors were modified from HIV, including HIV-1 vector system and HIV-2 vector system. HIV-1 is a double-stranded RNA virus whose genome structure is similar to other retroviruses, and contains genes encoding three main viral structural proteins, namely *gag, pol* and *env.* The *gag* gene encodes the core protein of the virus such as nucleocapsid protein (p7), inner membrane protein (p17) and capsid protein (p24), the *pol* gene encodes the enzyme related to viral replication, and the *env* gene encodes the viral envelope glycoprotein, which determines the targeting of the virus for infecting hosts. In addition, the viral genome also contains two regulatory genes *tat* and *rev. Rev* is mainly involved in the regulation of the expression level of a protein, and the protein encoded by *rev* can adjust the expression level of gag, pol, and env. The protein encoded by *tat* is involved in the control of RNA transcription, and binds to long terminal repeat (LTR) of the virus and then promotes the transcription of all genes of the virus. In addition, the genome also contains four accessory genes *vif*, *vpr*, *vpu* and *nef.* The genome structure of HIV-1 also contains other sequence structures required for the life history of the virus, such as the signals required for virus replication and packaging, *etc.* For example, the two ends of the genome are long terminal repeats, which contain *cis*-acting elements.

The establishment of the HIV-1 lentiviral vector system has undergone a gradual improvement process to gradually improve the biological safety of the vector.

The first-generation HIV-1 lentiviral vector is a two-plasmid system, including 3 structural genes *env*, *gag*, and *pol*, 2 regulatory genes *tat* and *rev,* and 4 accessory genes *vpr*, *vif*, *vpu*, and *nef*, has long terminal repeats (LTR) at both ends and a virus packaging signal sequence between 5' LTR and *gag.* The initial packaging of the lentiviral vector is to remove the trans-acting protein gene sequence in the HIV genome, and then package the nucleic acid vector containing the gene of interest and the packaging plasmid capable of trans-providing the proteins required by the virus particle, and co-transfect the host cell at the same time (such as 293T cells) for packaging. This is a replication-deficient vector system, which produces low titres of virus and can only infect CD4+ cells. During the transfection process, the packaged DNA for expressing a protein and vector DNA may be recombined, then there is a security risk of generating a virus system with replication ability.

The second-generation vector is a three-plasmid expression system, that is, the *cis*-acting sequence structures required for packaging, reverse transcription and integration and the sequences encoding trans-acting proteins in the HIV-1 genome are isolated, and then cloned into three independent vectors. The first one of the plasmids is a packaging plasmid, which contains a CMV promoter to control the expression of all of the virus structural genes except *env,* and the 3' LTR in the plasmid is replaced with the polyA of human insulin gene as a tail-adding signal. The second one of the plasmids is an envelope plasmid, which contains the sequence of the gene expressing the glycoprotein of vesicular stomatitis virus (VSV-G). This gene is used to replace the *env* gene of the original virus, and the product of this gene can increase the host range of the virus. The third one of the plasmids is a vector plasmid, which contains the gene sequence structure of interest to researchers and all related *cis*-acting elements. However, this three-plasmid system still retains the accessory genes of HIV-1. This plasmid system is more likely to produce active viruses due to accidents, so it is considered to have a lower safety factor.

The third-generation plasmid system removes all accessory gene sequences of the HIV virus. The removal of these accessory genes does not affect the titre and transfection ability of the virus, and at the same time increases the safety of the vector.

The fourth-generation vector is a four-plasmid system, which is also a widely used lentiviral vector system. On the basis of the third-generation, a self-inactivating lentiviral vector is constructed, that is, the 3' LTR of the U3 region is deleted, so that the vector lost the HIV-1 enhancer and promoter sequences, and RNA could not be transcribed even if all viral proteins are present. In addition, the *env* gene is placed on a separate expression plasmid, and the *tat* gene is removed and replaced with a heterologous promoter sequence. In this way, a system of four plasmids is formed, that is, pGag/Pol, pRev, and pVSV-G, and a nucleic acid vector in which the gene sequence of interest can be placed. The possibility of accidental production of active viruses in this system is greatly reduced. Such vector system may contain an inducible gene, the most representative one is the tetracycline-inducible system, which is the combination product of the regulatable system and the lentiviral vector, can artificially control the expression of the gene of interest introduced into the lentivirus, makes conditional expression and gene knockout possible, and continues to retain the advantage of self-inactivation (Meng F et al., (2014) Advances of lentiviral vectors. Zhongguo Fei Ai Za Zhi 17(12):870-6; Martinez-Molina, E. et al., (2020) Large-Scale Production of Lentiviral Vectors: Current Perspectives and Challenges. Pharmaceutics 12:1051).

Other types of lentiviral vector systems mainly include a simian immunodeficiency virus (SIV) vector system, a feline immunodeficiency virus (FIV) vector system, an equine infectious anemia virus (EIAV) vector system, a goat arthritis-encephalitis virus (CAEV) vector system, a bovine immunodeficiency virus (BIV) vector system, a sheep myelin shedding virus (VMV) vector system, *etc.*

### Preparation of viral vectors

The preparation of viral vector particles usually requires the following steps:
1) culturing and expanding a host cell; 2) introducing a viral vector expression system into the host cell; 3) culturing and expanding the host cell into which the viral vector expression system has been introduced; 4) collecting and purifying the viral vector produced.

Although it is now possible to package some viral vectors (such as AAV vectors) *in vitro* (cell-free), this packaging system still requires cell extracts, and the packaging efficiency is quite low, far from the level of production. So far, the packaging of viral vectors has mainly been carried out in host cells susceptible to the virus. Host cells not only provide the environmental conditions for virus replication and packaging, but also have many cellular components directly involved in the process of virus replication and packaging.

Host cells required for viral vector production are also known as production/packaging cell strains. In the context of lentiviral vectors, the term "packaging cell strain" may refer to a cell strain having introduced *gag* and *pol* and *env* genes; "Production cell strain" may refer to a cell strain having introduced *gag*, *pol*, *env* and exogenous genes. In one embodiment, "packaging cell strain" is a cell strain with *gag, pol* and *env* genes inserted into the cell genome; "production cell strain" is a cell strain in which *gag*, *pol*, *env* and exogenous genes are inserted into the cell genome. In another embodiment, "packaging cell strain" is a cell strain in which *gag, pol* and *env* genes are stably inserted into the cell genome; "production cell strain" is a cell strain in which *gag*, *pol*, *env* and exogenous genes are stably inserted into the cell genome.

The culture of host cells is divided into adherent culture and suspension culture. To increase the production of viral vectors, the total surface area available for cell attachment can be increased, or cell suspension culture can be carried out.

A HEK293 cells, also known as human embryonic kidney cell 293, is the most commonly used cell for the production of a retrovirus and other viral vectors. HEK293T cells having the SV40 T antigen integrated are able to produce higher viral titres than the mother cell strain HEK293. HEK293T cells are usually adherently cultured in a cell culture flask containing 10% fetal bovine serum medium, and the virus yield is small. To increase the surface area to which cells can attach, multilayer containers such as HYPERFlask^{™} (Corning), and multilayer cell factories such as the cell factory system (Numc, EasyFill, ThermoScientific, Waltham, MA, USA) can be used. HYPERFlask^{™} (Corning) has a total growth area of approximately 1720 cm² and has a gas permeable surface for oxygen and carbon dioxide exchange, and the yield is about 10 times that of T-150 culture flask. Cell factory systems (Numc, EasyFill, ThermoScientific, Waltham, MA, USA) can have up to 40 layers, which can produce a culture surface area equivalent to that of about 170 T-150 culture flasks. In addition, fixed-bed bioreactors such as iCELLis (Pall Life Sciences, Hoegaarden, Belgium) and the Scale-X^{™} bioreactor system (Univercells, Gosselies, Belgium) have been developed on the market. The fixed-bed is filled with polyethylene terephthalate (PET) fibres or of PET fibre layers, providing a large surface area for cell attachment. The cell growth area provided by iCELLis 500 is equivalent to about 3000 HYPERFlasks.

Although there are various equipment updates that can expand the culture of serum-dependent adherent HEK293T, it is still time-consuming and labour-intensive. Therefore, researchers began to try to adapt HEK293 cells to suspension culture. HEK293E cells are first adapted and cultured in suspension in serum-free medium. Since then, new such cell strains have been continuously developed, such as HEK293SF-3F6, *etc.* (Ansorge, S.et al., (2009) Development of a Scalable Process for High-Yield Lentiviral Vector Production by Transient Transfection of HEK293 Suspension Cultures. J. Gene Med. 11:868-876). Compared with adherent culture, suspension culture has many advantages, such as no need for complicated culture equipment, no mechanical force or enzymes to detach cells, more convenient for passaging, and convenient for scale-up.

For R&D enterprises of cell and gene therapy, the demand for viral vectors is limited during preclinical research, IND declaration and clinical trials, so almost all of the enterprises use transient transfection of adherent HEK293T cells to produce lentiviral vectors. The adherent culture of HEK293T cells is usually in the form of a cell factory, which mainly relies on increasing the number of culture units to increase the amount of cells. Therefore, when a large number of viral vectors is required, it often needs to occupy a large space, and the production labour is also large. To obtain large growth areas while reducing labour, one approach is to adapt a lentivirus-producing cell strain to suspension culture. Compared with adherent culture, suspension culture can greatly increase cell density.

The second step is to introduce the viral vector expression system into a host cell, that is, to transfect the host cell with a packaging nucleic acid vector, an enveloped nucleic acid vector, an accessory nucleic acid vector, a nucleic acid vector carrying a heterologous gene, *etc.*

"Viral expression system" in the present application refers to a system of one or more polynucleotides which, when introduced into a suitable host cell, is sufficient to support viral replication and packaging. Viral expression vectors can also include nucleic acid vectors carrying heterologous genes, and the encoded nucleic acids such as RNA or polypeptides/proteins can be packaged together into viral vector particles. For example, the expression system of lentivirus usually includes *gag-pol* gene, *env* gene, accessory gene *rev*, *etc.* In one embodiment, the lentiviral expression system comprises a nucleic acid vector comprising HIV-1 *gag-pol-rev* gene and a nucleic acid sequence encoding vesicular stomatitis virus glycoprotein (VSV-G) or a derivative protein thereof. In another embodiment, the lentiviral expression system comprises HIV-1 *gag-pol* gene, HIV-1 *rev* gene, and a nucleic acid sequence encoding vesicular stomatitis virus glycoprotein (VSV-G) or a derivative protein thereof. An AAV expression system typically includes a polynucleotide encoding AAV *rep* and *cap*, an accessory gene, and the rAAV genome. Herein, "nucleic acid vector" refers to a DNA or RNA vector, which may be a plasmid, or a bacterial artificial chromosome (BAC), a yeast artificial chromosome (YAC), a P1-derived artificial chromosome (PAC), fosmid or cosmid *etc.* in a specific embodiment. The nucleic acid vector may contain an origin of replication, a promoter, a transcriptional regulatory element, and the like as necessary. Any element can be operably linked to a promoter so that the expression can be controlled. The promoter mentioned herein may include known promoters (complete or partial), which may be constitutively active or inducible, for example, in the presence of a regulatory protein. In one embodiment, the nucleic acid vector additionally comprises a high-efficiency promoter, such as a CMV promoter. This promoter has the advantage of promoting high level expression of an element encoded on a non-mammalian nucleic acid vector. In another embodiment, the CMV promoter comprises a sequence derived from the human cytomegalovirus strain AD169. The sequence is available at Genome Accession No. X17403, for example from base pairs 173731 to 174404. In the present application, a nucleic acid vector carrying an exogenous gene may be referred to as a transfer vector, and in one embodiment may be a transfer plasmid. The transfer vector plasmid typically contains a promoter (such as CMV), a 3' LTR (which may or may not be a self-inactivating (*i.e.*, SIN) 3'-LTR), a 5' LTR (which may or may not contain the U5 region), an encapsidation sequence (ψ) and a potential exogenous gene linked to the promoter.

"Transfection" or "infection" as described herein refers to the introduction of an exogenous genetic material, such as the "viral expression system" described herein, into a host cell. Commonly used transfection methods known to those skilled in the art include, but are not limited to, the use of physical methods (for example, electroporation, cell extrusion, sonoporation, optical transfection, protoplast fusion, impact transfection, magnetofection, genetic gun or particle bombardment), chemical reagents (for example, calcium phosphate, highly branched organic compounds, or cationic polymers), or cationic lipids (for example, lipofection). Many transfection methods require contacting cells with a solution of plasmid DNA, and the cells grow and are selected for expression of a mark gene.

It is not easy to introduce an exogenous nucleic acid or a nucleic acid vector into a host cell, it is necessary to cross the barrier of cell membrane, and high transfection efficiency is necessary to produce a virus vector particle with high titre. The method of viral plasmid transfection can be roughly divided into two kinds, chemical methods and physical methods. Commonly used chemical methods include calcium phosphate co-precipitation, polyethyleneimine (PEI)-mediated transfection, Lipofectamine (Invitrogen)-mediated transfection, *etc.*, and the physical methods include electroporation, such as flow electroporation, microinjection, gene gun, *etc.*

Calcium phosphate co-precipitation is suitable for a variety of cell types, enables a large number of cells to be transfected at the same time, and can be used for large-scale virus preparation. However, calcium phosphate is very sensitive to pH changes and is quite toxic to cells. To avoid toxicity, host cells need to be cultured in a medium containing serum or albumin, and the medium should be replaced in time after transfection.

PEI-mediated transfection is as efficient as calcium phosphate. When using this method, it is necessary to select an appropriate PEI/DNA ratio. PEI is also toxic to cells, but it is also possible to leave the medium unchanged after transfection. Furthermore, the PEI method is not sensitive to pH. The PEI method can be used for both adherent and suspension cultured cells, and serum in the medium is essential (Toledo, J.R. et al., (2009) Polyethylenimine-Based Transfection Method as a Simple and Effective Way to Produce Recombinant Lentiviral Vectors. Appl. Biochem. Biotechnol. 157:538-544).

Lipofectamine-mediated transfection is the most efficient and convenient and suitable for a variety of cell types, and the transfection rate is very high, for example, 100% of the cells can be transfected with lentivirus in some instances. However, the use of Lipofectamine is cost-prohibitive for large-scale viral vector preparation.

Flow electroporation is as efficient as the calcium phosphate method, but requires only one-third or less of the amount of DNA, is not toxic to cells, and is suitable for cells in suspension culture.

In addition, addition of sodium butyrate to the medium after transfection has been reported to increase virus titres.

Among the above transfection methods, the PEI is more suitable for adherently cultured cells, the flow electroporation is more suitable for suspension cultured cells, and the calcium phosphate and the Lipofectamine are more suitable for small-scale applications due to cost reasons.

Once inside the host cell, the nucleic acid vector would be randomly integrated into the endogenous genome of the mammalian host cell. Therefore, it may also be desirable to select a host cell into which the nucleic acid encoded on the nucleic acid vector has been integrated, for example, using an antibiotic resistance selectable marker such as a zeocin resistance marker, *etc.*

Those skilled in the art would be aware of a method to facilitate integration of a nucleic acid vector, for example, linearizing the nucleic acid vector, if the nucleic acid vector, such as a plasmid, is naturally circular. The nucleic acid vector may additionally comprise a region having shared homology with the endogenous chromosome of the host cell to direct integration into a selected site in the endogenous genome. In addition, if recombination sites are present on the nucleic acid vector, they can be used for targeted recombination. Other methods of targeted integration are well known in the art. For example, methods that induce targeted cleavage of genomic DNA can be used to promote targeted recombination at selected chromosomal loci. These methods typically involve the use of an engineered cleavage system to induce double-strand breaks (DSBs) or nicks in the endogenous genome to repair the break by natural processes (such as non-homologous end joining) or to repair the break using a repair template (*i.e.*, homology directional restoration or HDR). By using specific nucleases such as engineered zinc finger nucleases (ZFNs) and transcriptional activators such as effector nucleases (TALENs), using the CRISPR/Cas9 system and engineered crRNA/tracr RNA (`single guide RNA') to direct specific cleavage and/or use nucleases based on the Argonaute system (for example from *T. thermophilus*, called `TtAgo', Swarts DC et al., (2014) DNA-guided DNA interference by a prokaryotic Argonaute. Nature. 507(7491):258-261), cleavage can occur. Targeted cleavage using one of these nuclease systems can utilize HDR or NHEJ-mediated processes to insert a nucleic acid at a specific target location. Therefore, in one embodiment, the nucleic acid sequence encoded on the nucleic acid vector is integrated into the genome *(i.e.,* the endogenous chromosome) of the host cell using at least one nuclease, wherein the at least one nuclease cleaves the genome of the host cell, allowing the integration of the nucleic acid sequence into the genome of the cell. In another embodiment, the at least one nuclease is selected from zinc finger nuclease (ZFN), TALE nuclease (TALEN), CRISPR/Cas nuclease system and a combination thereof.

Transfection is divided into transient transfection and stable transfection. In the case of transient transfection, the exogenous gene is expressed but not integrated into the genome of the cell and therefore not replicated. Transiently expressed exogenous genes in cells are expressed for a limited period of time, usually only for a few days, until the exogenous genes are lost due to various reasons during cell division. Stable transfection is based on transient transfection, but requires an important accidental process, that is, in a small number of transfected cells, the exogenous gene can be integrated into the genome of the cell. A hallmark of a stably transfected cell is that the exogenous gene becomes part of the genome of the cell and thus replicates. Progeny cells of stably transfected cells also express the exogenous gene, thus forming a stable cell strain.

Transient transfection generally lasts for several days. Transient transfection is used to study gene expression. Cells are usually harvested within 24 hours to 96 hours after transfection, and the specific time depends on various factors such as cell type and vector construction. For this reason, transient transfection is generally used to study short-term expression of a gene or a gene product, gene knockout or RNA-mediated gene silencing, and small-scale protein synthesis. Transient mRNA transfection yields results faster than traditional plasmid DNA transfection because mRNA can be expressed directly outside the nucleus, and in some systems mRNA can be expressed within minutes after transfection. Correspondingly, if long-term gene expression is required, stable transfection must be performed, such as in the case of large-scale protein synthesis, long-term pharmacological research, gene therapy research, and long-term genetic regulatory mechanism research, *etc.* Stable transfection takes longer and is more laborious than transient transfection.

Stably transfected cell strains are further divided into constitutive stably transfected cell strains and inducible stably transfected cell strains. The constitutive stably transfected cell strains usually do not express viral envelopes with higher cytotoxicity, such as lentivirus VSV-G, and produce viral vector particles at higher titres for several months. There are very few such cell strains, and the known cell strains stably transfected with lentivirus include STAR cell strain, WinPac cell strain, RD-MolPack cell strain, LentiPro26 cell strain, *etc.* The inducible stably transfected cell strains usually induce the expression of the viral envelope when the virus needs to be produced, for example, when an inducer such as an antibiotic is added to the growth medium. The inducible stably transfected cell strains can also produce viruses at high titres over a long period of time. For example, ProSavin is able to generate a titre of up to 3 × 10⁵ TU/ml in 83 days (Broussau, S. et al., (2008) Inducible Packaging Cells for Large-Scale Production of Lentiviral Vectors in Serum-Free Suspension Culture. Mol. Ther. 16:500-507; Stewart, H.J. et al., (2011) A Stable Producer Cell Line for the Manufacture of a Lentiviral Vector for Gene Therapy of Parkinson's Disease. Hum. Gene Ther. 22:357-369); Such a cell strain developed by Milani *et al* is able to generate a titre of 4.4 × 10⁶ TU/ml (Milani, M. et al., (2017) Genome Editing for Scalable Production of Alloantigen-free Lentiviral Vectors for in Vivo Gene Therapy. EMBO Mol. Med. 9:1558-1573).

Since it is difficult to obtain stably transfected cell strains, the current mainstream virus preparation such as lentiviral vector preparation still uses the HEK293T cell strain suitable for suspension culture.

The third step of viral vector preparation is to culture and expand the host cells into which the viral vector expression system has been introduced, and is similar to the first step.

The fourth step is to isolate and purify the viral vector. The collected cell culture supernatant containing viral vector particles may contain a variety of impurities, such as host cells, other proteins produced by host cells, plasmid DNA, serum, *etc.* The serum components are complex and may contain endotoxin and immunogenic protein, *etc.* These impurities need to be removed and the final product need to be concentrated. In these treatments, the biggest problem is how to maintain the activity/functionality of the viral vector, therefore, it is necessary to use as few steps as possible to complete the treatments in period of time as short as possible. The methods used for small-scale purification are obviously not suitable for large-scale production. Moreover, some purification steps, such as centrifugation, do not comply with cGMP regulations, and high-speed centrifugation would destroy the viral envelope to a certain extent.

Existing methods for large-scale purification of viral vectors include clarification, concentration, and purification.

Clarification refers to the removal of host cells and host cell debris after collection of the supernatant. Small-scale clarification can be carried out by methods such as centrifugation and microfiltration, while large-scale clarification preferably uses 45 µm porous membranes. To avoid pore clogging, membranes with decreasing pore size can be used to improve filtration efficiency.

Clarified viral vector particles can be concentrated using tangential flow filtration (TFF) and permeabilized into a suitable buffer, including the use of membranes with 1 to 100 nm pore size. This technology can greatly reduce the volume in a short time to concentrate virus particles, remove serum, degrade DNA fragments, *etc.* Most importantly, the operation with TFF is fully compliant with cGMP regulations. Since the liquid in TFF operation is parallel to the filter, the membrane clogging problem often encountered in other ultrafiltration methods can be greatly reduced, and the liquid flow rate can be kept at a high level. By TFF, approximately 90-100% of lentiviral particles can be recovered. Currently, TFF can process approximately 100 L of viral products (Valkama, A.J. et al., (2020) Development of Large-Scale Downstream Processing for Lentiviral Vectors. Mol. Ther. Methods Clin. Dev. 17:717-730).

For pharmaceutical products used in human, lentiviral vector products also need to be chromatographically purified. Anion exchange chromatography (AEX) is an efficient tool for the purification of viral vectors, especially lentiviral vectors, based on the positive charge of lentiviral vectors at neutral pH. When the viral vector supernatant is passed through a column with a negatively charged matrix, the positively charged viral particles bind to the negatively charged matrix, and impurities flow directly through the column. Afterwards, the viral vectors are exposed to a high-salt environment (0.5-1 M NaCl), and the bound particles are eluted from the anion-exchange column. High salt may inactivate virus particles, which is the only drawback of the technology. Exposure of virus particles to a high-salt eluent at room temperature inactivates 50% of the viruses (Segura, M.D.L.M. et al., (2005) A Novel Purification Strategy for Retrovirus Gene Therapy Vectors Using Heparin Affinity Chromatography. Biotechnol. Bioeng. 90:391-404).

Affinity chromatography is a purification technique used to separate biomolecules based on the specific interaction between target molecules and ligands attached to a chromatography column. Due to its high molecular selectivity, this technique can simplify the purification steps to a certain extent. Affinity chromatography can be divided into several categories according to the interaction, such as affinity chromatography based on hydrogen bonding, electrostatic interaction, van der Waals force, antibody-ligand interaction, *etc.* The antibody-ligand interaction is the most selective, and then affinity chromatography of antibodies binding to envelope proteins has not been used for virus vector purification. Heparin is a relatively cheap affinity ligand that interacts with a variety types of viruses and has been used in the purification of lentiviral vectors. In the purification, up to 53% of virus vectors can be recovered, and up to 94% of protein impurities and 56% of residual DNA can be removed. For the vectors and heparin, there is an interaction between positively charged molecules on the surface of virus particles and negatively charged heparin, so NaCl is usually used to elute the vectors from heparin column. The salt concentration typically needs to reach about 0.5 M to elute the viral vectors. This would require an additional cleaning or purification step to remove the salt, such as an additional TFF step. Studies have shown that heparin does not interact with viral envelope proteins and thus can bind to multiple types of viruses without loss in yield.

In large-scale purification of viral vectors, size exclusion chromatography (SEC) can be used as a clean-up step to efficiently remove remaining impurities. SEC, also known as gel filtration chromatography, is used for viral vector purification based on the difference between the large volume of virus particles and the small volume of impurities. All impurities are smaller than the pore size and pass through the column and only the virus particles with large volume retain. The disadvantage of SEC is that it is difficult to scale up, and the loading volume is only 10% of the column volume. In addition, SEC purification requires a linear low flow rate and a long operating time.

Following the purification step, lentiviral vectors should be frozen and stored at -80°C. The half-life of lentiviral vectors has been reported to be between 1 and 2 days at room temperature and about 8 days at 4°C (Higashikawa, F. et al., (2001) Kinetic Analyses of Stability of Simple and Complex Retroviral Vectors. Virology 280:124-131) and lentiviral vectors can be stored at -80°C for 1-2 years (Valkama, A.J. et al., (2020) Development of Large-Scale Downstream Processing for Lentiviral Vectors. Mol. Ther. Methods Clin. Dev. 17:717-730). Cryoprotectant replacement is performed before freezing. Sucrose and magnesium chloride can reduce the loss of functional virus particles and increase storage stability (Valkama, A.J. *et al.,* (2020) *supra*; Bandeira, V. et al., (2012) Downstream Processing of Lentiviral Vectors: Releasing Bottlenecks. Hum. Gene Ther. Methods 23:255-263; Kumru, O. S. et al., (2018) Physical Characterization and Stabilization of a Lentiviral Vector Against Adsorption and Freeze-Thaw. J. Pharm. Sci. 107: 2764-2774).

The present application specifically relates to a method for preparing a virus vector particle by using the cell strain of the present application, including PowerS^{™}-293T, and the method comprises the following steps:
i) culturing the cell strain;
ii) introducing a virus vector expression system into the cell strain;
iii) culturing the cell strain under a condition favourable for the production of the viral vector particle; and
vi) collecting the viral vector particle.

Step i) may include suspension culture of the cell strain. In one embodiment, the cell strain is cultured with shaking in serum-free medium under the condition of 37°C and 8%°C. The serum-free medium can be selected from LV-MAX production medium, Expi293 expression medium and OPM-293 CD03 medium.

Step i) may also include passaging of the cell strain.

Step ii) may include introducing the viral expression system into the cell strain via a physical or chemical transfection. The physical transfection can include electroporation, microinjection, gene guns, *etc.* The chemical transfection method can include calcium phosphate co-precipitation, polyethyleneimine (PEI)-mediated transfection, Lipofectamine (Invitrogen)-mediated transfection, *etc.*

The viral vector expression system can comprise a packaging nucleic acid vector and an envelope nucleic acid vector. The viral vector expression system can also comprise an accessory nucleic acid vector. The viral vector expression system can also comprise a nucleic acid vector containing a heterologous nucleic acid, such as a transfer nucleic acid vector containing a heterologous nucleic acid. These nucleic acid vectors can be plasmids.

The heterologous nucleic acid is no greater than 4 kb in length. The heterologous nucleic acid may encode a polypeptide or a protein such as a chimeric antigen receptor, or a nucleic acid such as siRNA. In one embodiment, the heterologous nucleic acid may encode a chimeric antigen receptor (CAR), such as a CAR targeting CD19, CD20, CD22, CD30, CD33, or CD269 (BCMA). In one embodiment, the CAR encoded by the heterologous nucleic acid can target an antigen involved in growth and differentiation signalling, including carcinoembryonic antigen (CEA), epidermal growth factor receptor (EGFR, HER2), tyrosine kinase receptor (EPHA2 ), *etc.* In one embodiment, the CAR encoded by the heterologous nucleic acid can target an antigen involved in tumour angiogenesis, including vascular endothelial growth factor (VEGF), VEGF receptor (VEGFR), *etc.* In one embodiment, the CAR encoded by the heterologous nucleic acid can target a tumour interstitial and an extracellular matrix antigen of the tumour support structure, including fibroblast activation protein (FAP), tenascin protein, *etc.* The plasmids containing the heterologous nucleic acid can include pCDH, pCMV, *etc.*

The viral vector may be a retroviral vector, an adenoviral vector or an adeno-associated viral vector. The retroviral vector may be a lentiviral vector, an alpha-retroviral vector, a gamma-retroviral vector, or a foamy retroviral vector. In one embodiment, the retroviral vector is a lentiviral vector selected from HIV-1, HIV-2, SIV, FIN, EIAV and VISNA. In one embodiment, the lentiviral vector may be an HIV-1 vector. In another embodiment, the viral vector may be an adeno-associated viral vector, such as AAV2, AAV5, AAV8, AAV9, and AAV10.

The retroviral vector expression system may comprise, a vector containing retroviral structural protein gag-pol gene, and a nucleic acid vector containing env gene or a functional substitute thereof. The env gene or the functional substitute thereof can encode vesicular stomatitis virus glycoprotein (VSV-G) or a derivative protein thereof. The retroviral vector expression system may also comprise a nucleic acid vector containing an accessory gene *rev* or a similar gene. The retroviral vector expression system can also comprise a nucleic acid vector containing a heterologous nucleic acid, such as a transfer nucleic acid vector containing a heterologous nucleic acid.

In one embodiment, the method is used to prepare a lentiviral vector, and comprises introducing three nucleic acid vectors into the cell strain. The three nucleic acid vectors comprise HIV-1 gag-pol-rev gene, a nucleic acid encoding vesicular stomatitis virus glycoprotein (VSV-G) or a derivative protein thereof, and a heterologous nucleic acid, respectively.

In one embodiment, the method is used to prepare a lentiviral vector, and comprises introducing four nucleic acid vectors into the cell strain. The four nucleic acid vectors comprise HIV-1 gag-pol-rev gene, a HIV-1 rev gene, a nucleic acid encoding vesicular stomatitis virus glycoprotein (VSV-G) or a derivative protein thereof, and a heterologous nucleic acid, respectively. The nucleic acid vector containing the heterologous nucleic acid may contain a self-inactivating lentiviral long terminal repeat.

Step iii) may include suspension culture of the cell strain. The cell strain can be cultured under any condition suitable for the cell strain to produce the viral vector, and the condition can be determined by those skilled in the art. In one embodiment, the cell strain is cultured with shaking in serum-free medium under the condition of 37°C and 8%°C. The serum-free medium can be selected from LV-MAX production medium, Expi293 expression medium and OPM-293 CD03 medium.

Step iii) may also include passaging the cells.

The present application has carried out small-scale and pilot-scale production of PowerS^{™}-293T host cells containing a viral vector expression system, such as 300 ml shake flask culture, such as suspension culture in a 1 L, 2 L, 10 L, 50 L and 200 L bioreactor (such as a WAVE reactor or a glass reactor) to produce the viral vector.

The method may also comprise determining the titre of the viral vector after step vi).

### Application of viral vectors

A viral vector expression system may comprise a nucleic acid vector containing a heterologous nucleic acid, such as a transfer vector plasmid containing a heterologous nucleic acid, and the heterologous nucleic acid is used for encoding a therapeutic or research protein such as a chimeric antigen receptor, or a therapeutic or research nucleic acid such as siRNA. The application of viral vectors mainly depends on the protein and nucleic acid encoded by the heterologous nucleic acid.

The heterologous nucleic acid can encode a therapeutic molecule, such as a therapeutic peptide, or a therapeutic nucleic acid such as siRNA. A "therapeutic molecule" may be a peptide or protein that mitigates or alleviates symptoms resulting from protein deficiency or defect in a cell or subject. Alternatively, the "therapeutic" peptide or protein encoded by the heterologous nucleic acid is a substance that confers a benefit on a subject, such as correcting a genetic defect, correcting a gene (expression or function) defect, or providing an anticancer effect. The therapeutic nucleic acid can include, for example, siRNAs, antisense molecules, and miRNAs. The heterologous nucleic acid can encode a number of useful products.

The heterologous nucleic acid can encode hormones and growth and differentiation factors, including but not limited to insulin, glucagon, growth hormone (GH), parathyroid hormone (PTH), growth hormone releasing factor (GRF), follicle stimulating hormone (FSH), luteinizing hormone (LH), human chorionic gonadotropin (hCG), vascular endothelial growth factor (VEGF), angiopoietins, angiostatin, granulocyte colony stimulating factor (GCSF), erythropoietin (EPO), connective tissue growth factor (CTGF), basic fibroblast growth factor (bFGF), acidic fibroblast growth factor (aFGF), epidermal growth factor (EGF), transforming growth factor alpha (TGF-alpha), platelet-derived growth factor (PDGF), insulin-like growth factors I and II (IGF-I and IGF-II), any of the transforming growth factor beta superfamily, including TGFβ, kinetin, and inhibitors, or any of the bone morphogenetic proteins (BMPs) BMP1-15, nerve growth factor (NGF), brain-derived neurotrophic factor (BDNF), neurotrophins NT-3 and NT4/5, ciliary neurotrophic factor (CNTF), glial cell-derived neurotrophic factor (GDNF), aggregin, netrin-1 and netrin-2, hepatocyte growth factor (HGF), kainic acid, noggin, and tyrosine hydroxylase.

Other useful heterologous nucleic acid products include proteins that modulate the immune system, including but not limited to cytokines and lymphokines, for example thrombopoietin (TPO), interleukins (IL) IL-1 to IL-17, monocyte chemoattractant protein, leukemia inhibitory factor, granulocyte-macrophage colony-stimulating factor, Fas ligand, tumour necrosis factor alpha and beta, interferon alpha, beta and gamma, stem cell factor, flk-2/fFlt3 ligand, immunoglobulin IgG, IgM, IgA, IgD and IgE, chimeric immunoglobulin, humanized antibody, single-stranded antibody, T cell receptor, chimeric T cell receptor, single chain T cell receptor, G protein-coupled receptors (GPCRs) for example CCR5, Class I and Class II MHC Molecules, and engineered immunoglobulin and MHC molecules. Useful heterologous nucleic acid products also include regulatory proteins such as complement regulatory proteins, membrane cofactor proteins (MCPs), decay accelerating factors (DAFs), CR1, CF2, and CD59.

Other useful heterologous nucleic acid products include those gene products that can correct congenital metabolic defects, including but not limited to carbamyl synthetase I, ornithine transcarbamylase, arginosuccinate synthetase, arginosuccinate lyase, arginase, fumarylacetacetate hydrolase, phenylalanine hydroxylase, alpha-1-antitrypsin, glucose-6-phosphatase, porphobilinogen deaminase, clotting factor, for example Factor V, Factor VIIa, Factor VIII, Factor IX, Factor X, Factor XIII or protein C, cystathione beta-synthetase, branched chain ketoacid decarboxylase, albumin, isovaleryl-CoA dehydrogenase, propionyl-CoA carboxylase, methylmalonyl-CoA mutase, glutaryl-CoA dehydrogenase, Insulin, β-glucosidase, pyruvate carboxylate, hepatic phosphorylase, phosphorylase kinase, glycine decarboxylase, H protein, T protein, cystic fibrosis transmembrane regulator (CFTR) sequence and dystrophin cDNA sequence.

Other useful heterologous nucleic acid products include those that can make up for functional or activity defects, absence or deletion, for example antibodies, Retinal pigmented epithelial cell specific 65 kDa protein (RPE65), erythropoietin, low-density lipoprotein receptor, lipoprotein lipase, ornithine transcarbamylase, β-globulin, α-globulin, spectrin, α-anti-trypsin, adenosine deaminase (ADA), metal transporter (ATP7A or ATP7), sulfamidase, enzymes involved in lysosomal storage diseases (ARSA), hypoxanthine guanine phosphoribosyltransferase, beta-25 glucocerebrosidase, sphingomyelinase hexosaminidase, lysosomal hexosaminidase, branched chain ketoacid dehydrogenase, hormones, growth factors (for example, insulin-like growth factor 1 and 2, platelet-derived growth factor, epidermal growth factor, nerve growth factor, neurotrophins 3 and 4, brain-derived neurotrophic factor, glial cell-derived growth factor, transforming growth factor α and β, *etc.),* cytokines (for example alpha interferon, beta interferon, interferon gamma, interleukin 2, interleukin 4, interleukin 12, granulocyte macrophage colony-stimulating factor, lymphotoxin, *etc.*), products of suicide genes (for example, herpes simplex virus thymidine kinase, cytosine deaminase, diphtheria toxin, cytochrome P450, deoxycytidine kinase, tumour necrosis factors, *etc.),* drug resistance proteins (for example, used in cancer therapy to confer drug resistance), tumour suppressor proteins (for example p53, Rb, Wt-1, NF1, VonHippel-Lindau (VHL), adenomatous polyposis (APC)), immunomodulatory peptide, tolerance or immunogenic peptides or proteins Tregitopes, or hCDR1, insulin, glucokinase, guanylate cyclase (LCA-GUCY2D), Rab guard protein 1, LCA5, ornithine ketoacid aminotransferase, Retinoschisis 1 (X-linked retinoschisis), USH1C (Usher syndrome 1C), X-linked retinitis pigmentosa GTPase (XLRP), MERTK (AR form of RP: retinitis pigmentosa), DFNB1 (Connexin26 deafness), ACHM2, 3 and 4 (colorblind), PKD-1 or PKD-2 (polycystic kidney disease), TPP1, CLN2, genetic defects caused by lysosomal storage diseases (for example, sulfatase, N-acetylglucosamine-1-phosphotransferase, Cathepsin A, GM2-AP, NPC1, VPC2, sphingolipid activator proteins, *etc.),* one or more zinc finger nucleases for genome editing, or donor sequences used as repair templates for genome editing.

The heterologous nucleic acid may also encode a tumor-associated antigen (TAA). Non-limiting examples of TAA include: testicular tumor-associated specific antigens (for example, MAGE, BAGE, and GAGE), melanocyte differentiation antigens (for example, tyrosinase, Melan-A/MART-1), CDK4, MUM-1, β-catenin, gp100/pmel17, TRP-1, TRP-2, MITF, MITF-A, and MITF-M. Non-limiting examples of additional TAAs include melanoma GP75, Annexin I, Annexin II, adenosine deaminase binding protein (ADAbp), PGP9.5 (Rode et al. 1985. Histopathology 9:147), Colorectal-associated antigen (CRC)-C017-1A/GA733, Ab2BR3E4, CI17-1A/GA733, Hsp70, Hsp90, Hsp96, Hsp105, Hsp110, HSPPC-96, stress protein gp96, gp96-associated cellular peptide, G250, dipeptidyl peptidase IV (DPPIV), thyroglobulin, STn, carcinoembryonic antigen (CEA), carcinoembryonic antigen (CEA) peptide CAP-1, carcinoembryonic antigen (CEA) peptide CAP-2, etv6, aml1, prostate specific antigen (PSA), PSA antigenic determinant PSA-1, PSA-2, PSA-3, Ad5-PSA, parathyroid hormone-related protein (PTH-rP), EGFR, PLU1, carcinoembryonic antigen-immature laminin receptor (OFA-iLR), MN/CAIX (CA9), HP59, cytochrome oxidase 1, sp100, msa, RanGTPase activating protein, Rab-GAP protein, PARIS-1, T cell receptor/CD3-ζ chain, cTAGE-1, SCP-1, glycolipid antigen-GM2, GD2 or GD3, GM3, FucosylGM1, glycoprotein antigen-Tn, Sialyl-Tn, TF and Mucin-1, CA125 (MUC-16), MAGE family antigen, GAGE-1, 2, BAGE, RAGE, LAGE-1, GnT-V, MUM-1, EP-CAM/KSA, CDK4, MUC family antigen, HER2/neu, ErbB-2/neu, p21ras, RCAS1, α-fetoprotein, E-cadherin, α-catenin, β-catenin and γ-catenin, NeuGcGM3, Fos-related antigen, cyclophilin B, RCAS1, S2, L10a, L10a, telomerase rt peptide, cdc27, p120ctn, PRAME, GA733/EoCam, NY-BR-1, NY-BR-2, NY-BR-3, NY-BR-4, NY-BR-5, NY-BR-6, NY-BR-7, NY-ESO-1, L19H1, MAZ, PINCH, PRAME, Prp1p/Zer1p, WT1, adenomatous polyposis coli protein (APC), PHF3, LAGE-1, SART3, SCP-1, SSX-1, SSX-2, SSX-4, TAG-72, TRAG-3, MBTAA, Smad tumour antigen, lmp-1, HPV-16E7, c-erbB-2, EBV encoded nuclear antigen (EBNA)-1, herpes simplex virus thymidine kinase (HSVtk), alternative splicing isoforms of XAGE-1, TGFbetaRII frameshift mutation, and BAX frameshift mutation.

In addition, the heterologous nucleic acid can encode gene product, for example CAIX, CD19, CD20, CD22, CD30, CD33, CD44v7/8, CEA, EGF-RIII (epidermal growth factor receptor type mutant 3) EGP-2, erb-B2, erb-B2, 3, 4, FBP, fetal acetylcholine receptor, GD2, Her2/neu, IL-13R-a2, KDR, κ light chain, LeY, L1 cell adhesion molecule, MAGE-A1, Mesothelin, MUC1, NKG2D, carcinoembryonic antigen (h5T4), PSCA, prostate specific membrane antigen (PSMA), prostatic acid phosphatase (PAP), prostate epithelial cell-derived Ets transcription factor (PDEF), TAA-targeting mAbIgE, TAG-72 and VEGF-R2.

Alternatively, the heterologous nucleic acid can include, for example, siRNAs, antisense molecules, and miRNAs. Clinically useful lentiviral vectors can also express antisense genes against human immunodeficiency virus (HIV) (Levine BL et al., (2006) Gene transfer in humans using a conditionally replicating lentiviral vector. Proc Natl Acad Sci U S A. 103(46):17372-7) and other important human pathogens. Naldini (Naldini L. (2011) Ex vivo gene transfer and correction for cell-based therapies. Nat. Rev. Genet. 12:301-315) discusses and cites these and other useful applications of lentiviruses and related vectors. The antisense gene contained in the lentiviral vector inhibit the expression of: Huntington's (HTT) gene, a gene associated with dentatorubral-pallidoluysian atrophy (for example atrophin 1, ATN1); X-chromosomal androgen receptor in spinobulbar muscular atrophy, human Ataxin-1, -2, -3 and -7, (CACNA1A) encoded Cav2.1P/Q voltage-dependent calcium channel, TATA-binding protein, Ataxin8 opposite chain also known as ATXN8OS, β isoform of serine/threonine protein phosphatase 2A55kDa regulatory subunit B in spinocerebellar ataxia (type 1, 2, 3, 6, 7, 8, 12, 17), FMR1 in fragile X syndrome (fragile X mental retardation 1), FMR1 in fragile X-related tremor/ataxia syndrome (fragile X mental retardation 1), FMR1 (fragile X mental retardation 2) or AF4/FMR2 family member 2 in fragile X mental retardation; myotonic protein kinase (MT-PK) in myotonic dystrophy; frataxin in Friedrich's ataxia; a mutations in the superoxide dismutase 1 (SOD1) gene in amyotrophic lateral sclerosis; a gene involved in the pathogenesis of Parkinson's and/or Alzheimer's disease; Apolipoprotein B (APOB) and subtilisin precursor protein convertase/kexin type 9 (PCSK9), hypercholesterolemia; HIVTat and human immunodeficiency virus trans-activating factor of transcribed genes in HIV infection; HIVTAR, HIVTAR and human immunodeficiency virus trans-activating factor response element genes in HIV infection; C-C chemokine receptor (CCR5) in HIV infection; Rous sarcoma virus (RSV) in RSV infection, liver-specific microRNA (miR-122) in hepatitis C virus infection; p53, acute kidney injury, or renal transplantation with delayed graft function, or renal injury with acute renal failure; protein kinase N3 (PKN3) in progressive or metastatic solid tumours; LMP2; LMP2 is also known as proteasome subunit beta type 9 (PSMB9), metastatic melanoma; LMP7, also known as proteasome subunit beta type 8 (PSMB8), metastatic melanoma; MECL1, also known as proteasome subunit beta type 10 (PSMB10), metastatic melanoma; vascular endothelial growth factor (VEGF) in solid tumours; spindle kinesin in solid tumours, apoptosis suppressing B-cell CLL/lymphoma in chronic myeloid leukemia (BCL-2), nucleotide reductase M2 (RRM2) in solid tumours, Furin in solid tumours; Polo-like kinase 1 (PLK1) in liver tumours, diacylglycerol acyltransferase 1 (DGAT1) in hepatitis C virus infection, β-catenin in familial adenomatous polyposis; beta 2-adrenergic receptors, glaucoma; RTP801/Redd1, also known as DNA damage-induced transcription factor 4 protein, in diabetic macular edema (DME) or age-related macular degeneration; vascular endothelial growth factor receptor I (VEGFR1) in age-related macular degeneration or choroidal neovascularization, caspase 2 in non-arteritic ischemic optic neuropathy; keratin 6AN17K mutant protein in pachyonychia congenita; influenza A virus A genome/gene sequence in influenza infection; severe acute respiratory syndrome (SARS) coronavirus genome/gene sequence in SARS infection; respiratory syncytial virus infection genome/gene sequence in respiratory syncytial virus infection; Ebola filovirus genome/gene sequence in Ebola virus infection; hepatitis B and C virus genome/gene sequence in hepatitis B and C virus infection; herpes simplex virus (HSV) genome/gene sequence in HSV infection, Coxsackievirus B3 genome/gene sequence in Coxsackievirus B3 virus infection; torsin-like A (TOR1A) silencing pathogenic allele (allele-specific silencing) in primary dystonia, pan-class I and HLA allele in transplantation; mutated rhodopsin gene (RHO) in autosomal dominant hereditary retinitis pigmentosa (adRP); or inhibitory nucleic acids that bind to transcripts of any of the aforementioned genes or sequences.

In one embodiment, the heterologous nucleic acid may encode a chimeric antigen receptor (CAR), such as a CAR targeting CD19, CD20, CD22, CD30, CD33, or CD269 (BCMA). In one embodiment, the CAR encoded by the heterologous nucleic acid can target an antigen involved in growth and differentiation signalling, including carcinoembryonic antigen (CEA), epidermal growth factor receptor (EGFR, HER2), tyrosine kinase receptor (EPHA2 ), *etc.* In one embodiment, the CAR encoded by the heterologous nucleic acid can target an antigen involved in tumour angiogenesis, including vascular endothelial growth factor (VEGF), VEGF receptor (VEGFR), *etc.* In one embodiment, the CAR encoded by the heterologous nucleic acid can target a tumour interstitial and an extracellular matrix antigen of the tumour support structure, including fibroblast activation protein (FAP), tenascin protein, *etc.* The plasmids containing the heterologous nucleic acid can include pCDH, pCMV, *etc.*

### Screening of host cell strains

Selecting a suitable host cell can greatly increase the production of viral vectors. Preferred cells are those suitable for serum-free culture, and/or adapted for suspension culture. As mentioned above, if a serum-free medium is used for cell culture and expansion, potential immunogenic substance contamination and animal-derived components in the final product can be reduced, and the downstream purification process can be simplified, and the simplification of the purification process can improve the yield and activity of viral vectors. In addition, suspension culture is more suitable for large-scale production of viral vectors than adherent culture.

### Serum-free culture

Generally speaking, the growth of animal cells depends on the presence of serum. Most cells cannot proliferate without the addition of serum. Serum medium is usually supplemented with bovine serum.

The composition of serum is complex, including proteins that assist in the transportation of substances, nutrients, hormones and factors that promote cell growth, *etc.* Because of the complexity of the natural components of serum, serum plays a variety of functions in the process of cell culture, including providing hormones to maintain cell growth and promoting the growth of cultured cells; supplementing nutrients that are absent or in a small amount in the basal medium; providing binding proteins to promote cell recognition and utilization of vitamins, lipids and other hormones; in some cases, the binding proteins can bind to toxic metals and pyrogens to detoxify; promoting cell adherence and providing factors that can promote cell spreading on a plastic culture matrix; acting as a pH buffer; and providing protease inhibitors to inactivate residual trypsin during cell digestion and protect cells from damage. However, the use of bovine serum has the risk of contaminating exogenous viruses and becoming a pathogenic factor, and due to the inconsistency of biological activities and factors between different batches of bovine serum, the reproducibility of products and experimental results is poor, and the residual bovine serum in products is also easy to cause allergic reactions in virus vector recipients to the serum.

Serum-free medium, referred to as SFM, is a cell culture medium without adding serum. It is the third type of medium after natural medium and synthetic medium. Compared with a traditional medium, the serum-free medium does not contain animal serum or other biological extracts, but it can still maintain the growth and reproduction of cells *in vitro* for a long time. Due to its relatively clear composition and simple preparation process, SFM has been widely used in the field of modern biotechnology, and it is also a powerful tool for elucidating basic research issues of cell growth, proliferation, differentiation, and gene expression regulation.

There are currently two types of serum-free media, one does not contain any added components of animal origin, and the other one does not contain any unspecified added components. At present, the following four serum-free media are widely used.

The first one is the serum-free medium in the general sense, which uses various biological materials that can replace the function of serum to prepare cell culture medium, such as bovine serum albumin, transferrin, insulin and other biological macromolecules, and protein-free mixed lipids extracted from serum and hydrolysed proteins. Its characteristic is that the protein content in the medium is relatively high, but the chemical composition of the added substances is not clear, and it contains a large amount of animal-derived proteins.

The second one is an animal source-free medium. The animal source-free medium developed by many commercial companies is based on safety considerations for the production of recombinant drugs. The added components in the culture medium do not have components of animal origin, and the required proteins are derived from recombinant proteins or protein hydrolysates. Such components can ensure the needs of cell growth and proliferation.

The third one is an animal protein-free medium. The medium does not use animal-derived proteins at all, but there are still some added components derived from hydrolysed fragments of plant proteins or synthetic polypeptide fragments and other derivatives. Such a medium has a relatively stable composition, but must be supplemented with steroid hormones and lipid precursors, and is highly specific to the cells being cultured.

The fourth one is a chemically defined medium. Such a medium is currently the safest and most ideal medium, and can ensure the consistency between batches of medium, and the added small amount of animal-derived protein hydrolysates and proteins are all defined components. Its characteristic is that the nature of the culture medium is determined, and it is convenient to formulate the culture medium.

The advantages of the serum-free medium include avoiding quality variation between serum batches and improving the reproducibility of cell culture and experimental results; avoiding the toxic effect of the serum on cells and serum-derived contamination; avoiding the influence of serum components on experimental research; conducive to the differentiation of cultured cells *in vitro*; improving the expression level of the product and making the cell product easy to purify; having a stable composition and production in large quantities; containing no mitogen inhibitors, and promoting cell proliferation, *etc.* The disadvantage of such a medium is that cells are susceptible to certain mechanical and chemical factors in the serum-free medium, and the preservation and application of the medium are not as convenient as the traditional synthetic medium; high cost; very specific, that is, one serum-free medium is only suitable for the culture of a certain type of cells; cells at different differentiation stages of development require different formulations, and the selection of growth factors and cytokines is particularly important. Moreover, while removing the serum, the protective effect provided by some serum proteins is also removed, so the requirements for the purity of reagents and water and instrument cleanliness are high.

The serum-free medium is made from a nutrient-complete basal medium supplemented with hormones, growth factors, adherence factors, and binding proteins.

The basal medium used in cell culture in the early days includes natural medium such as plasma clot, lymph fluid, soybean peptone and embryo extract. In 1950, Morgan *et al.* developed 199 medium on the basis of previous researches, which marked a new stage of animal cell culture medium and the stage of synthetic culture medium. The synthetic medium is a basal medium composed of a certain proportion of amino acids, vitamins, inorganic salts, glucose, *etc.* according to the needs of cell growth. At present, there are hundreds of synthetic media commercially available on the market. Among many synthetic media, MEM, DMEM, RPMI 1640, F12, and TC100 are the most widely used. The composition of the basal medium is well known, so when culturing different cell strains, some components of the basal medium can be adjusted accordingly to better meet the nutritional requirements of the cell strains or increase the expression of proteins of interest.

Added factors in the serum-free medium, also known as supplementary factors, are the general term that replace various serum factors. Most serum-free culture media must be supplemented with 3 to 8 factors, and any single factor cannot replace serum. More than 100 such factors are known, some of which are essential supplementary factors, such as insulin, sodium selenite, and transferrin, and most of the others are accessory factors. According to different functions, supplementary factors can be divided into four categories.

The first category of the supplementary factors are hormones and growth factors. Hormones, such as insulin, growth hormone and glucagon, need to be added when many cells are cultured in the serum-free medium. Almost all cell strains require insulin, which is a polypeptide that can bind to an insulin receptor on a cell to form a complex, promote the synthesis of RNA, proteins and fatty acids, and is an important cell survival factor. In the serum-free culture of cells, the concentration of insulin used is 0.1-10 µg/ml. Jan *et al.* believed that the rapid depletion of insulin in batch culture is the main reason for the decrease of cell specific growth rate. In addition, thyroxine and steroid hormones such as progesterone, hydrocortisone, and estradiol are also commonly used supplementary factors for serum-free cell culture. Different cell strains require different types and quantities of hormones. Growth factors are essential supplementary factors to maintain the survival, proliferation and differentiation of cultured cells *in vitro.* According to their chemical properties, they can be divided into polypeptide growth factors and steroid growth factors. The growth factors added to the serum-free medium are mainly polypeptide growth factors. In recent years, 20-30 kinds of polypeptide growth factors have been identified, and for more than half of them, corresponding recombinant growth factors can be obtained through genetic recombination. The common growth factors in the serum-free medium include epidermal growth factor (EGF), fibroblast growth factor (FGF) and nerve growth factor (NGF). Growth factors are potent mitogens that shorten cell population doubling time.

The second category of the supplementary factors are binding proteins. There are two binding proteins, one is transferrin and the other one is albumin. There are specific transferrin receptors on most mammalian cells. The binding of the receptors and the complex of transferrin and iron ions is the main source for cells to obtain the essential trace element iron. In addition, transferrin also has the properties of growth factors and can bind to other trace elements such as vanadium. Different cells need different amounts of transferrin. Albumin is also a commonly used added factor in the serum-free media. It can bind with vitamins, lipids, hormones, metal ions and growth factors to stabilize and regulate the activity of the above substances in the serum-free medium, in addition it can bind to toxins and reduce the influence of proteases on cells.

The third category of the supplementary factors are adherence factors. Most eukaryotic cells require adherence to a suitable substrate for growth *in vitro.* Cell adherence is a complex process involving the adsorption of adherence factors on the surface of a vessel or carrier, the binding of cells with the adherence factors, *etc.* Adherence factors commonly used in serum-free culture include intercellular substances and serum components, such as fibronectin, collagen, laminin, polylysine, *etc.* In the suspension culture as described in the present application, for example, adherence factors do not need to be added to the culture medium.

For serum-free culture of some cell strains, it is necessary to supplement some low molecular weight chemicals, such as trace elements, vitamins, lipids, *etc.* Vitamin B mainly participates in cell metabolism in the form of coenzyme, and vitamin C and vitamin E have antioxidant effects. Butanediamine and linoleic acid provide lipids needed for cell membrane synthesis and water-soluble lipids needed for cell growth.

At present, the serum-free medium has been widely used in large-scale animal cell culture. In the application field of biological products such as vaccine growth, monoclonal antibodies and various biologically active proteins, optimizing the composition of the serum-free medium can enable different cells to maintain high-density culture in an environment that is most conducive to cell growth and expression of products of interest, thereby reducing manufacturing cost. During human cell culture, the use of the serum-free media can also selectively control and avoid the overgrowth of fibroblasts. Under serum-free culture conditions, the growth of certain cells and the production of antibodies are even several times higher than those with serum.

In addition to the production of biological products, the serum-free medium is also widely used in the fields of cell biology, pharmacology, and oncology. For example, the serum-free medium can be used to study the differentiation conditions of cells. The composition of the serum-free medium can be well known chemical substances, so the types and quantities of important biologically active substances can be increased or decreased according to the needs of research. This provides an effective means for studying the conditions of cell differentiation. Another example is that the serum-free medium can be used to select target cells from mixed culture of a variety of cell. By selecting certain components in serum-free culture, the excessive growth of non-target cells in primary tissue culture can be inhibited, and the purpose of selecting target cells can be achieved. Serum-free culture is also used in the study of tumour pathology and etiology. For example, it is used to study the influence of carcinogens on cells, to study the ability of tumour cells to respond to peripheral signals that may trigger the terminal differentiation of normal cells, to study the relationship between the growth and migration of normal cells and tumour cells and basement membrane signals, *etc.* In addition, the serum-free culture can also be used to study the interaction of hormones, growth factors and drugs with cells.

Commercially available serum-free cell growth media include FreeStyle^{™} 293 (Gibco^{™}, Life Technologies), DMEM/F12 (Gibco^{™}, Life Technologies), SFM4Transfx-293 (HyClone^{™}, ThermoScientific), CDM4HEK293 (HyClone ^{™}, ThermoScientific), StemPro-34SFM (Gibco^{™}, Life Technologies), FreeStyle F17 (Gibco^{™}, Life Technologies), 293SFMII (Gibco^{™}, LifeTechnologies), CD293 (Gibco^{™}, LifeTechnologies), LV-MAX (A35834-01, Gibco^{™}), Expi293 (Gibco ^{™}, A14351-01), EX-CELL^{®}293 (Sigma, 14571C-1000mL), BanlanCD HEK293 (Irvine, 91165), Pro293s-CDM (Lonza, 12-765Q), OPM-293 CD03 (Shanghai OPM Biosciences Co., Ltd., 81070-001), and Celkey CD HEK293 (JSBiosciences, 10804-19008) medium.

### Suspension culture

Suspension culture refers to dispersing and suspending cells in the culture medium for the culture of single cells and small cell clusters and is a culture method of adherence-independent cells. Certain adherence-dependent cells can also be cultured by this method after adaptation and selection. Scaling up the suspension culture is relatively simple, simply by increasing the volume. When the depth of the culture medium exceeds a certain value, it is necessary to shake the culture medium by a shaking or rotating device. When the depth of the culture medium is deeper, carbon dioxide and oxygen need to be introduced to ensure sufficient gas exchange.

Suspension culture process is divided into suspension cell culture process and adherent cell microcarrier suspension culture process according to cell adherence. Suspension cells (CHO cells, BHK21l cells, *etc.)* can be directly produced and proliferated in a reactor, the cells grow freely, the culture environment is uniform, the sampling is simple, the culture operation is simple and controllable, the scaling-up is convenient, the contamination rate and the cost are low; however, the suspension culture of adherent cells in the reactor requires the use of microcarriers, and the nutrient environment at the contact site between the cells and the spheres is poor, and the culture, sampling for observation and scaling-up processes are complex and costly. Although compared with suspension culture, the microcarrier culture process is complicated, but it still has great advantages compared with roller culture, and can improve production scale, product quality and labour efficiency, so it is the main means of suspension culture of adherent cells.

The cell suspension culture process is divided into batch culture, fed-batch culture and perfusion culture according to the culture method. The batch culture can intuitively reflect the growth and metabolic changes of cells in a bioreactor, and the operation is simple, but there are many metabolic waste products in the initial stage, which inhibits cell growth and the cell growth density is not high; the fed-batch culture is simple to operate, has high yield, is easy to scale up, and is widely used, but it is necessary to design a fed-batch culture medium; The culture volume of the perfusion culture is small, the recovery volume is large, the residence time of the product in a tank is short, and the product can be recovered in time and stored at a low temperature, which is beneficial to maintain the activity of the product; but the operation is complicated, the utilization efficiency of the cell culture medium is low, and the rotary filter is easy to block, *etc.* For different viral vectors, different cell culture methods are used. For example, for a biological product that is secreted and whose activity decreases rapidly, the perfusion culture should be used, and the perfusion culture is also more suitable for microcarrier culture.

After selecting a suitable production process, process control becomes the key. In order to ensure that the cells grow in an optimal environment, it is necessary to use the online monitoring function of the reactor to control various operating parameters. The cells are sensitive to temperature, and appropriate heating or cooling methods are required to control the culture temperature at 35°C to 37°C to avoid cell damage. The suitable pH range for animal cell growth is generally between 6.8 and 7.3, and any pH lower than 6.8 or higher than 7.3 may have adverse effects on cell growth.

Whether the scale of cell culture can be enlarged is an important prerequisite for selecting a reactor. The scaling up of the suspension culture is mainly through increasing the volume of the reactor or increasing the number of reactors. Increasing the volume of the reactor can save a lot of supporting engineering and personnel costs, while multiple small reactors, although flexible in operation, are relatively expensive. In the production of biological products abroad, large-scale bioreactors that can achieve scaling-up step by step are widely used. The selection and configuration of bioreactors also need to consider and meet the production process and capacity requirements. The standardization of the reactor interface, the speed of supply of accessories, and the quality of after-sales service should all be considered factors in the selection of bioreactors for industrialization, so as to avoid delays in production.

In the process of liquid suspension culture, attention should be paid to the subculture of cells in time, because when the cells grow to a certain period, they would enter the stationary phase of division. For most suspension cultures, the cells reach their maximum density on days 18 to 25, at which time the first subculture should be performed. When subculturing, large cell clumps and inoculum debris should be removed.

### Screening of cell strains

The essence of cell screening and adaptation is to apply modern cell biology techniques to perform study on adapting to different culture modes (such as suspension) and different media (such as serum-free media), reducing cell apoptosis rate, improving cell vitality, prolonging cell life cycle and increasing product concentration, so as to screen a cell strain suitable for production. In order to achieve a stable adaptation, the high density cell culture is usually shifted to low density cell culture, and the high concentration serum culture is gradually shifted to low serum concentration culture. Because cells are difficult to repair after damage, the cell viability should be kept more than 90% during adaptation. The proliferative ability of cells directly affects the production capacity, and the determination of their proliferative ability should be maintained during adaptation. When cells are adapted, their expression and secretion ability should also be determined to avoid changes in the expression characteristics of the cells after adaptation. The proliferative ability, vitality and production ability of the adapted cells should be able to meet the needs of large-scale industrial production. Due to the different nutritional requirements of specific cells, the degree of difficulty in achieving adaptation is also different. During adaptation, it is necessary to select a suitable cell culture medium and supplement certain nutrients in a targeted manner to meet the specific needs of cells so that the adapted cells can keep their characteristics of suspension or serum-free growth.

Also, in suspension culture, cells tend to clump. Cell clumping prevent many of the plasmids used for vector production from transfecting cells, and cells in the middle of large clumps die from lack of nutrients. At present, the method of improving cell clumping is mainly by adding an anti-clumping agent. The addition of the anti-clumping agent may significantly reduce the transfection efficiency, resulting in cells that cannot be transfected with packaged lentiviruses.

There is an urgent need in the field for an efficient and convenient method for adapting/screening a cell strain with high dispersibility, high proliferation density, and high production of viral vectors.

In the present application, the inventors mainly improved the adaptation/screening method for HEK293T cells, which are currently the most used in lentivirus preparation.

First of all, the inventors does not adopt the culture step of gradually reducing the serum concentration from high concentration serum culture commonly used in the field, but directly add the adherent cells cultured in an ordinary medium to a serum-free medium for suspension culture, which greatly saves time and may also be helpful for successfully screening a cell strain with high dispersibility (that is, not easy to clump), high proliferation density and high virus vector production.

In addition, the inventors of the present application also fine that using several specific serum-free suspension media, such as LV-MAX production medium (Gibco, A35834-01), Expi293 expression medium (Gibco, A14351-01) and OPM-293 CD03 medium (Shanghai OPM Biosciences Co., Ltd., 81070-001), is more conducive to the screening of a cell strain with high dispersibility and without clumping. Specifically, the cells screened from these three media can reach a high density (~1E7 cells/ml) and the proportion of clumped cells is relatively low, which is conducive to the screening of subclones without clumping.

The present application screens mainly HEK293T cells, which are commonly used in the preparation of lentiviral vectors, have fast proliferation, high transfection efficiency, and efficient production of viral vectors, and have the potential for large-scale production of viral vectors.

Specifically, the present application provides a method for screening or adapting HEK293T cell strains suitable for serum-free suspension culture, comprising:
i) taking a HEK293T cell adherently cultured in a serum-containing medium and placing the cell in a serum-free medium for suspension culture, and
ii) selecting a monoclonal cell strain suitable for serum-free suspension culture.

The serum-containing medium in step i) may contain 10% serum, such as 10% fetal bovine serum.

The suspension culture in step i) may include shaking culture at 37°C and 8% CO₂.

The suspension culture in step i) may include cell passaging.

The serum-free medium can be selected from LV-MAX production medium (Gibco, A35834-01), Expi293 expression medium (Gibco, A14351-01), FreeStyle^{™} 293 expression medium (Gibco, 12338-018), CD293 AGT medium (Gibco, 11913-019), EX-CELL^{®}293 serum-free medium (Sigma, 14571C-1000mL), BanlanCD HEK293 medium (Irvine, 91165), Pro293s-CDM serum-free medium (Lonza, 12-765Q), OPM-293 CD03 medium (Shanghai OPMBiosciences Co., Ltd., 81070-001) and Celkey CD HEK293 medium (JSBiosciences, 10804-19008), preferably LV-MAX production medium, Expi293 expression medium and OPM-293 CD03 medium.

Step ii) may include selecting a monoclonal cell strain with high dispersibility (that is, non-clumping) and suitable for serum-free suspension culture.

Step ii) may include culturing the cell in a semi-solid medium, selecting a single cell from the semi-solid medium, and performing suspension culture on the cell.

The suspension culture of the selected cell in step ii) may include shaking culture at 37°C and 8% CO₂. The suspension culture of the selected cell in step ii) may include cell passaging.

The semi-solid medium may be a methylcellulose-based medium supplemented with a screening medium.

During the implementation of this method, no anti-clumping agent needs to be added.

In the method of the present application, 1) the cells adherently cultured in the serum-containing medium are directly subjected to serum-free suspension culture, and there is no step of progressive serum decreasing, and the screening process can be can shortened; 2) the serum-free medium can be used to culture the cells, reduce potential immunogenic substance contamination and animal-derived components, and simplify the downstream purification process; 3) the serum-free medium selected from LV-MAX production medium, Expi293 expression medium and OPM-293 CD03 medium is used, and is more conducive to screening the HEK293T cell strain suitable for suspension culture, that is, with high proliferation density and high dispersibility; 4) the use of the anti-clumping agent is avoided, and the transfection efficiency of the cells is not affected; and 5) the use of the semi-solid medium facilitates the screening of qualified single clones.

### Semi-solid culture

In addition, the inventors of the present application used the semi-solid culture method for monoclonal cell screening instead of the limiting dilution method in the above screening method.

The limiting dilution method is a cell cloning method routinely used in laboratories. However, the limited dilution method may add multiple cells in one well, so multiple subcloning is required to increase the probability of monoclonality. In addition, it was found that when the limited dilution method is used to screen subclones, the individual cells in the well plate could not proliferate normally, which may be related to the characteristics of the cells themselves. The semi-solid culture method is a faster and simpler cloning method. Individual cells are immobilized in a semi-solid medium and grow into discrete monoclonal cell populations, which is conducive to tracking the growth of cells. And when the monoclonal cells are expanded to a certain number and then transferred to the well plate, the problem that individual cells cannot proliferate normally can be improved. In addition, the semi-solid medium can be used to plant multiple cells in the same well, which can reduce the workload of operators, and compared with the limited dilution method, can reduce the number of culture well plates and save reagents, consumables and incubator space.

The semi-solid medium is mostly used in the field for the culture of hybridomas and CHO cells, so that cell cloning can be performed more efficiently. The use of semi-solid culture in the screening of viral vector packaging/production cells has not been reported yet.

### The host cell strain of the present application

Starting from the HEK293T cells purchased from ECACC, a variety of cell strains with high proliferation density and high dispersibility were obtained through the cell strain screening/adaptation method of the present application.

One of them, named PowerS^{™}-293T, was deposited at the China General Microbiological Culture Collection Centre (CGMCC) on December 7, 2020 according to the Budapest Treaty with a deposit number of CGMCC No.: 21110.

The PowerS^{™}-293T cell strain can grow in suspension culture in a serum-free medium. The serum-free medium can be selected from LV-MAX production medium, Expi293 expression medium and OPM-293 CD03 medium.

Cell clumping cannot appear during the suspension culture of the PowerS^{™}-293T cell strain.

The PowerS^{™}-293T cell strain can be used to produce a viral vector.

PowerS^{™}-293T cell strain can grow in suspension culture in the serum-free medium, with high proliferation density, good viability, and no cell clumping, that is, with high dispersibility. Therefore, no additional anti-clumping agent is needed in the suspension culture process.

The doubling time of PowerS^{™}-293T cells in OPM-293 CD03 medium is short, about 22 hours, and the cell density at logarithmic phase can reach 5-7 × 10⁶ cells/ml, and the highest density can reach 1.48 × 10⁷ cells/ml.

Compared with Gibco's CTS^{™} virus production cells (Pub. No. MAN0018590), PowerS^{™}-293T has a shorter cell doubling time and produces more viral vector particles after transfection with viral plasmids.

The technical solutions of the present invention are further illustrated in more detail by the examples and in conjunction with the accompanying drawings. Unless otherwise specified, the methods and materials in the examples described below are conventional products that can be purchased from the market. Those skilled in the art of the present invention would understand that the methods and materials described below are exemplary only and should not be considered as limiting the scope of the present invention.

### Example 1. Serum-free suspension adaption of HEK293T cells

The HEK293T cells purchased from ECACC were incubated in a 37°C water bath for less than 1 minute, and the cells in the cryovial were quickly thawed until there is no ice in the cryovial.

The cells were transferred from the cryovial to a sterile centrifuge tube, 4-5 ml of pre-warmed DMEM (containing 10% FBS) was added, and centrifuged at 200 g for 5 minutes.

The supernatant in the culture flask was discarded and the cell pellet was resuspended in 10 ml of DMEM (containing 10% FBS). The resuspended cells were added into a T75 culture flask, and the T75 culture flask was placed in a 37°C, 5% CO₂ incubator.

The cells were incubated for three days and the cell culture medium in the flask was discarded when the cells were > 90% confluent. The degree of cell confluency herein refers to the percentage of the surface area of the culture vessel covered by the monolayer of the cells observed under a microscope for the cells grown in a monolayer. The adherent layer was rinsed once with 5 ml of DPBS. The cells were incubated with 0.25% trypsin (Gibco, 12563-029) for 2 minutes, after the cells were detached, 4-5 ml of DMEM containing 10% FBS was added to the T75 flask culture.

The cells were transferred to a sterile centrifuge tube and centrifuge at 200 g for 5 minutes.

The supernatant was aspirated, the cell pellet was resuspended in 10 ml of DMEM (containing 10% FBS), the resuspended cells was added to a T75 culture flask, and the T75 culture flask was placed in a 37°C, 5% CO₂ incubator.

The cells were passaged when they were fully adapted for adherent growth and reached 90% confluency.

The cells were digested with 0.25% trypsin and centrifuged at 200 g for 5 minutes. The supernatant was aspirated and a fresh serum-free medium (SFM) was added to the cell pellet to resuspend the cells. There are 9 kinds of serum-free media, including LV-MAX production medium (Gibco, A35834-01), Expi293 expression medium (Gibco, A14351-01), FreeStyle^{™} 293 expression medium (Gibco, 12338-018), CD293 AGT medium (Gibco, 11913-019), EX-CELL^{®}293 serum-free medium (Sigma, 14571C-1000mL), BanlanCD HEK293 medium (Irvine, 91165), Pro293s-CDM serum-free medium (Lonza, 12-765Q), OPM-293 CD03 medium (Shanghai OPM Biosciences Co., Ltd., 81070-001) and Celkey CD HEK293 medium (JSBiosciences, 10804-19008). The cells in the serum-free medium were transferred to a 125 ml shake flask, then cultured in a 37°C, 8% CO₂ incubator on a shaker with a shaking diameter of 19 mm and a rotating speed of 125 rpm.

When the cell density reached 3-6 × 10⁶ cells/ml, the cells were subcultured, and the inoculated cells had a density of 0.35-0.55 × 10⁶ cells/ml, and incubated for 3-4 days until the viability exceeded 95%, wherein the viability was determined by VI-CELL cell counter (BECKMAN).

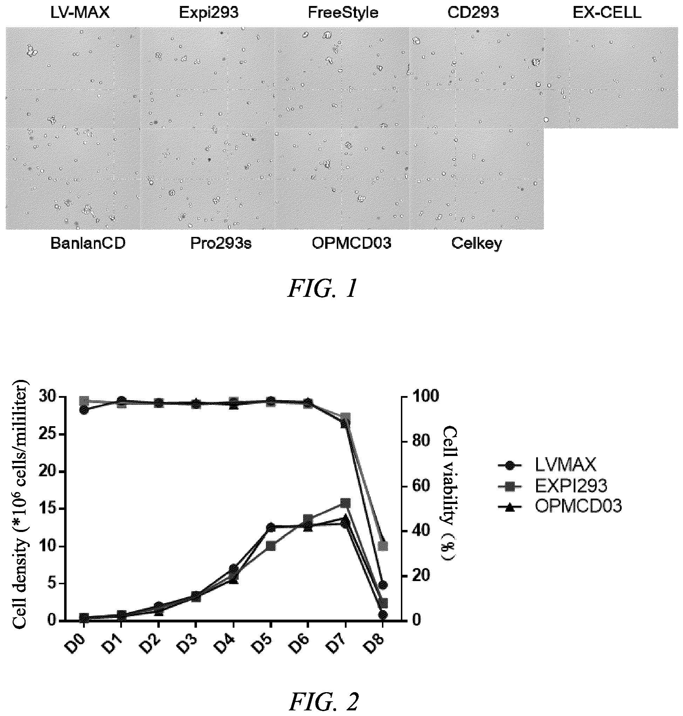

The cell liquid cultured in the 9 kinds of serum-free media was put into the cell culture plate, and a microscope was used to take pictures to observe the colony morphology, as shown in FIG. 1.

Among them, the cells adapted in LV-MAX production medium (Gibco, A35834-01), Expi293 expression medium (Gibco, A14351-01), and OPM-293 CD03 medium (Shanghai OPM Biosciences Co., Ltd., 81070-001) had higher density and better dispersibility. The cells adapted in these three media were taken to determine the growth curve. Specifically, the cells were inoculated into a cell culture flask at a cell density of 0.4 ± 0.5 × 10⁶ cells/ml, which was recorded as D0, and 600 microliters of cell suspension was aspirated for cell counting, then cultured for 8 days in suspension, and 600 µl of cell suspension was taken every day for cell counting, and the cell growth curve was drawn and shown in FIG. 2. FIG. 2 shows that the cell growth density and viability in the above three media have the same trend, the highest cell density is greater than 1 × 10⁷ cells/ml, and the cell viability is greater than 90%.

Cells at higher density can be obtained by passaging and selection, and finally the cell strain with the highest suspension density of 1.2 × 107 cells /ml in the above three media was selected. The cells were cryopreserved to a final density of 1 × 10⁷ viable cells/ml, and used as the selected cell strain suitable for serum-free suspension culture. At this time, 10-20 cells may aggregate.

### Example 2. Performance optimization of serum-free suspension adapted HEK293T

In order to further solve the problem of clumping of serum-free suspension adapted HEK293T cells, the cells adapted in LV-MAX production medium, Expi293 and OPM-293 CD03 medium were used for clonal screening using semi-solid Matrigel. The specific steps are as follows.

The serum-free suspension adapted cells obtained in example 1 were correspondingly diluted with the above three serum-free media to prepare a cell suspension with a density of 5 × 10³ cells/mL.

100, 200, 300, or 400 µL of each cell suspension was added to each 2.0 mL cryovial containing 1 mL of semi-solid Matrigel (Stemcell, 03816) to form a cell concentration gradient, that is, each cell had 4 cell gradients, and the total number of cells in each well was 500, 1000, 1500 and 2000, respectively. The lid was closed and the cryovials were shaken for 1-2 min. The medium was opaque. The culture medium was allowed to stand for 2-5 min, so that the culture medium was concentrated at the bottom of the cryovial and bubbles rised to the liquid level.

A 5 mL disposable syringe was used for inoculation. Specifically, 1-1.5 mL of the above semi-solid cell suspension was added to each culture well of a 6-well plate, and each density was repeated three times. The culture plate was slightly tilted and rotated to ensure that the culture medium was evenly distributed at the bottom of the culture well.

The covered 6-well plate was placed in a covered large Petri dish in which there was an uncovered 100 mm Petri dish filled with sterile water. Alternatively, the grooves between the wells of the culture plate were filled halfway up with sterile water or PBS before covering the 6-well plate. The colonies in culture were checked according to the doubling time of the cell strain. The colonies were visible to the naked eye and were typically 0.5 - 1.0 mm in diameter. The number of colonies grown at each inoculation density was counted. The culture plate was photographed to keep the colony morphology photos.

After 7 days, a 24-well plate was prepared, and the corresponding serum-free medium was added to each well plate at 1 mL per well. A 10 µL pipette tip was used to aspirate 5 µL of the serum-free medium in which the corresponding cells were adapted, the larger clones in the 6-well plate culture were gently aspirated, and the cells were transferred to a 24-well plate, and observed under the microscope whether they were single cells. The 24-well plate was placed in a 37°C, 8% CO₂ incubator, and cultured on a shaker with a rotation speed of 125 rpm and a shaking diameter of 19 mm.

The medium was supplemented once every 3 days, the cells were expanded into a 6-well plate with 2 mL medium/ well according to the number of cells, placed in a 37°C, 8% CO₂ incubator, and cultured on a shaker with a shaking diameter of 19 mm and a rotation speed of 125 rpm.

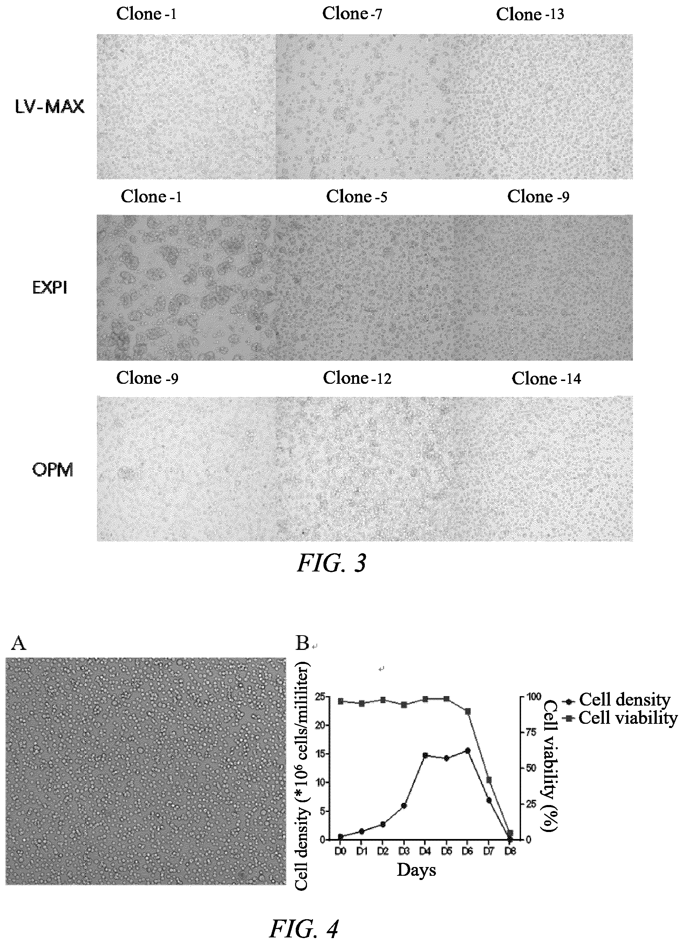

According to the number of cells, the cells were expanded into a 125 mL shake flask for 3 passages, and the cell suspension was taken and observed under a microscope. The cell morphology is shown in FIG. 3. As can be seen from FIG. 3, cell strains that did not clump can be screened out by cloning. The cells that did not clump were frozen and stored.

A cell strain obtained from OPM-293 CD03 adaption, named PowerS^{™}-293T, had a cell morphology as shown in FIG. 4A, and an 8-day growth curve and growth vitality graph as shown in FIG. 4B. The cell strain did not clump, the cell doubling time is short, about 22 hours, and the cell density at logarithmic phase can reach 5-7 × 10⁶ cells/ml, and the highest density can reach 1.48 × 10⁷ cells/ml.

In comparison, Gibco's CTS^{™} virus production cells (Pub. No. MAN0018590) have a doubling time of approximately 26 hours.

### Example 3. Preparation of viral vectors using serum-free suspension adapted HEK293T cells PowerS^{™}-293T

The cells PowerS^{™}-293T obtained in example 2 were inoculated into a 125 ml shake flask at 0.55 × 10⁶ cells/ml using OPM-293 CD03, and cultured for three days in a 37°C, 8%°C incubator on a shaker with a shaking diameter of 19 mm and a rotation speed of 125 rpm.

The cell suspension was taken from the culture shake flask containing the suspension cells, and the cells were counted, and transfected when the cell density reached 5.5 × 10⁶ cells/ml and the cell viability was greater than 95%.

Taking the 30 ml transfection system as an example, the transfection cell density was first adjusted, and the cell culture liquid (transfection volume (30 ml) × transfection cell density (5 × 10⁶ cells/ml)/actual viable cell density (the actual counted viable cell density of the cells on the day of transfection)) was taken and placed in 27 ml of OPM-293 CD03 medium. The cell culture flask was placed in a 37°C, 8%°C incubator, and subjected to suspension culture on a shaker with a shaking diameter of 19 mm and a rotation speed of 125 rpm. Before transfection, the cell density was adjusted to 5.5 × 10⁶ cells/ml, and the cell culture bottle was placed in the incubator for later use.

In the meantime, a transfection complex was prepared.

Specifically, the OPM-293 CD03 medium and PEIpro transfection reagent were shaken 4-5 times before use. 56.25 µg of plasmids in TE buffer was added to 1.5 ml of OPM-293 CD03 medium preheated at 37°C, and transfer plasmids: pMDLg.pRREKan (gag-pol, 8847 bp): pRSV-Rev-Amp (rev, 5779 bp): pMD2.gKan(vsvg, 4137 bp) = 4: 2: 2: 1 were gently pipetted and mixed 4-5 times, labelling as tube 1-DNA. Among them, there are three kinds of transfer plasmids, which were pCDH vectors which contained heterologous genes and had sizes of 7545 bp, 9517 bp and 9505 bp, respectively. 112.5 ml of PEIpro transfection reagent (Polyplus Transfection, PT-115-100) was added to 1.5 ml of OPM-293 CD03 medium preheated at 37°C, gently pipetted and mixed 4-5 times, labelling as tube 2-PEIpro. The transfection buffer in tube 2 was transferred into tube 1, pipetted repeatedly with a pipette for more than 8-10 times, vortexed with a vortexer for 10 seconds as soon as possible, and allowed to stand at room temperature for 15 minutes.

The obtained transfection complex was slowly added into the liquid containing cells to be transfected with shaking the flask gently while adding. After the operation was completed, the cell culture flask was placed in the incubator for suspension culture. Sodium butyrate was added at a final concentration of 5 mM 24 hours after transfection. After 48 hours, the virus was harvested and the virus titre was determined using FACS.

Gibco's CTS^{™} virus production cells (Pub. No. MAN0018590) were used as a positive control. Except for the transfection reagent, other operations were the same as those of PowerS^{™}-293T. Specifically, Gibco transfection used LV-MAX transfection kit (ThermoFisher, A35348).

The results of the transfection tests are shown in Table 1. The virus yield of PowerS^{™}-293T cells was higher than that of Gibco cells, and the virus titres after testing were all greater than 1 × 10⁷ TU/mL.

**Table 1. Transfection tests of PowerS^{™}-293T cells and Gibco cell strains with different shuttle plasmids**

| | Transfer plasmid 1-7545 bp (containing heterologous gene GFP) | | Transfer plasmid 2-9517 bp (containing heterologous gene 1) | | Transfer plasmid 3-9505 bp (containing heterologous gene 2) | |
|---|---|---|---|---|---|---|
| | PowerS^{™}-293T | Gibco | PowerS^{™}-293T | Gibco | PowerS^{™}-293T | Gibco |
| Titre (TU/ml) | 1.88E+08 | 1.3E+ 08 | 1.29E+07 | 1.01E +07 | 1.51E+07 | 8.32E +06 |

The embodiments of the present invention are not limited to the above-mentioned embodiments. Without departing from the spirit and scope of the present invention, those skilled in the art can make various changes and improvements to the present invention in forms and details, and all these changes and improvements are considered to fall into the protection scope of the present invention.

| | | |
|---|---|---|
| **0-1** | **Form PCT/RO/134 (SAFE) Indications Relating to Deposited Microorganism(s) or Other Biological Material (PCT Rule13bis)** | |
| 0-1-1 | Software version | **CEPCT** |
| | | **Version 10. 25. 41 (20201201) MT/FOP 20140331/0. 20. 5. 21** |
| **0-2** | **International Application No.** | PCT/CN2021/141958 |
| **0-3** | **Applicant's or agent's file reference** | **P10974PCT** |

| | | |
|---|---|---|
| **1** | **The indications made below relate to the deposited microorganism(s) or other biological material referred to in the description on:** | |
| **1-1** | **page** | **5** |
| **1-2** | **line:** | **33-35** |
| **1-3** | **Identification of deposit** | |
| 1-3-1 | Name of depositary institution | **China General Microbiological Culture Collection Centre** |
| 1-3-2 | Address of depositary institution | **No.3, No.1 Courtyard, Beichen West Road, Chaoyang District, Beijing 100101, China, Institute of Microbiology, Chinese Academy of Sciences** |
| **1-3-3** | **Date of deposit** | **07 December 2020 (07. 12. 2020)** |
| **1-3-4** | **Deposit Number** | **CGMCC No. 21100** |
| **1-4** | **Additional remarks** | |
| **1-5** | **Designated States for Which Indications are Made** | **All designations** |
| **1-6** | **Indications for separate submission** | |
| | These indications will then be submitted to the International Bureau | |

### FOR RECEIVING OFFICE USE ONLY

| | | |
|---|---|---|
| **0-4** | **This form was received with the international application:** | |
| | (yes or no) | |
| 0-4-1 | Authorized officer | |

### FOR INTERNATIONAL BUREAU USE ONLY

| | | |
|---|---|---|
| **0-5** | **This form was received by the international Bureau on:** | |
| 0-5-1 | Authorized officer | |

## Claims

1. A novel human embryonic kidney HEK293T cell strain, deposited at the China General Microbiological Culture Collection Centre with a deposit number of CGMCC NO.: 21100.

2. A method for screening a HEK293T cell strain suitable for serum-free suspension culture, **characterized in that** the method comprises:
i) taking a HEK293T cell adherently cultured in a serum-containing medium, and placing the cell in a serum-free medium for suspension culture; and
ii) selecting a monoclonal cell strain suitable for serum-free suspension culture.

3. The method of claim 2, **characterized in that** the serum-containing medium in step i) contains 10% serum.

4. The method of claim 2, **characterized in that** the suspension culture in step i) includes shaking culture at 37°C and 8% CO₂.

5. The method of claim 2, **characterized in that** the suspension culture in step i) includes cell passaging.

6. The method of claim 2, **characterized in that** step ii) includes selecting a non-clumping monoclonal cell strain suitable for serum-free suspension culture.

7. The method of claim 2 or 6, **characterized in that** step ii) includes culturing the cell in a semi-solid medium, selecting a single cell from the semi-solid medium, and performing suspension culture on the cell.

8. The method of claim 7, **characterized in that** the suspension culture of the selected cell in step ii) includes shaking culture at 37°C and 8% CO₂.

9. The method of claim 7, **characterized in that** the suspension culture of the selected cell in step ii) includes cell passaging.

10. The method of claim 7, **characterized in that** the semi-solid medium is a methylcellulose-based medium supplemented with a screening medium.

11. The method of claim 2, **characterized in that** the serum-free medium is selected from LV-MAX production medium, Expi293 expression medium and OPM-293 CD03 medium.

12. The method of any one of claims 2-11, **characterized in that** the method is carried out without the addition of an anti-clumping agent.

13. A cell strain screened by using the method of any one of claims 2-12.

14. The cell strain of claim 13, **characterized in that** the cell strain does not clump in a liquid medium.

15. Use of a medium selected from LV-MAX production medium, Expi293 expression medium and OPM-293 CD03 medium in screening a non-clumping HEK293T cell strain suitable for suspension culture.

16. A method for preparing a virus vector using the cell strain of any one of claims 1, 13 and 14, **characterized in that** the method comprises:
i) performing suspension culture on the cell strain;
ii) introducing a virus vector expression system into the cell strain;
iii) culturing the cell strain under a condition favourable for the production of the viral vector; and
vi) collecting the viral vector.

17. The method of claim 16, **characterized in that** step vi) is further followed by step v) determing the titre of the viral vector.

18. The method of claim 16, **characterized in that** the viral vector expression system comprises a packaging nucleic acid vector and an envelope nucleic acid vector.

19. The method of claim 16, **characterized in that** the viral vector expression system further comprises an accessory nucleic acid vector, and/or a nucleic acid vector containing a heterologous nucleic acid.

20. The method of claim 19, **characterized in that** the heterologous nucleic acid is not greater than 4 kb in length.

21. The method of claim 16, **characterized in that** the viral vector is selected from a retroviral vector, an adenoviral vector and an adeno-associated viral vector.

22. The method of claim 21, **characterized in that** the retroviral vector is a lentiviral vector.
